# EUROPÄISCHE PATENTSCHRIFT

(11) **EP 0 799 829 B1**
(45) Veröffentlichungstag und Bekanntmachung des Hinweises auf die Patenterteilung: **30.07.2003**
(21) Anmeldenummer: 97104941.6
(22) Anmeldetag: 24.03.1997
(51) Int. Cl.: C07D 487/04, C07D 471/04, C07C 69/616, C07C 69/612, C07C 69/65

(54) **Verfahren zur Herstellung von enantiomeren reinen Cycloalkano-indol- und azaindol- und pyrimido [1,2a] -indol-carbonsäuren und deren aktivierte Derivate**
Process for the preparation of enantiomerically pure cycloalkanoindole-, azaindole- and pyrimido [1,2a] indole carboxylic acids and their activated derivatives
Procédé pour la préparation de dérivés énantiomériques pure d'acides carboxyliques substitués par un groupe cycloalkanoindole-, azaindole- ou pyrimido [1,2a] indole et leur dérivés activés

(30) Priorität: 04.04.1996 DE 19613549
(43) Veröffentlichungstag der Anmeldung: 08.10.1997
(73) Patentinhaber: BAYER AG, 51368 Leverkusen (DE)
(72) Erfinder: Lenfers, Jan-Bernd, Dr., 42327 Wuppertal (DE); Fey, Peter, Dr., 42111 Wuppertal (DE); Naab, Paul, Dr., 42287 Wuppertal (DE); Van Laak, Kai, Dr., 51061 Köln (DE)

(56) Entgegenhaltungen:
- EP-A- 0 513 533
- EP-A- 0 560 163
- EP-A- 0 610 698
- EP-A- 0 705 831

## Beschreibung

Die Erfindung betrifft ein Verfahren zur Herstellung von enantiomerenreinen Cycloalkano-indol- und azaindol- und Pyrimido[1,2a]indolcarbonsäuren und deren aktivierte Derivate, die wichtige Zwischenprodukte zur Synthese von antiatherosklerotisch wirksamen Cycloalkano-indol- und azaindol- und Pyrimido[1,2a]-indol-derivaten darstellen.

Es ist bekannt, daß enantiomerenreine Cycloalkano-indol- und azaindol-carbonsäuren und deren aktivierte Derivate durch Diastereomerentrennung nach üblichen Methoden, beispielsweise durch Chromatographie oder fraktionierte Kristallisation, in die entsprechenden Enantiomeren getrennt werden können.

Dieses Verfahren hat mehrere Nachteile: Sowohl die chromatographische Diastereomerentrennung als auch die fraktionierte Kristallisation der Diastereomeren ist mit einem großen technischen Aufwand verbunden. Außerdem fallen hierbei in der Regel fünfzig Prozent des "falschen" Diastereomeren an, das dann nicht mehr in den ursprünglichen Herstellprozeß rückführbar ist.

Dieser fünfzig prozentige Ausbeuteverlust beeinträchtigt die Wirtschaftlichkeit eines (groß)technischen Verfahrens erheblich, ganz abgesehen davon, daß fünfzig Prozent "Nebenprodukt" zu entsorgen sind. Ferner sind die üblichen chiralen Hilfsreagenzien im allgemeinen bereits in kleinen Mengen sehr teuer und können dann meist nur über einen aufwendigen Syntheseweg hergestellt werden.

Es wurde nun gefunden, daß man enantiomerenreine Cycloalkano-indol- und azaindol- und Pyrimido[1,2a]indol-carbonsäuren und deren aktivierte Derivate der allgemeinen Formel (I) 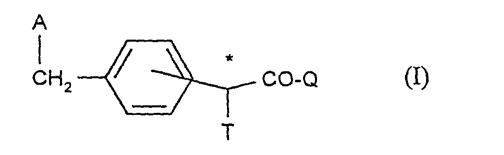 in welcher
- A: für einen Rest der Formel 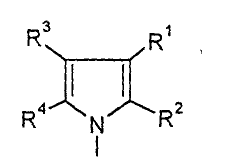 oder 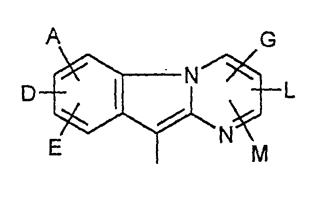 steht,
- A, D, E, G, L und M: gleich oder verschieden sind und Wasserstoff, Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen bedeuten, das seinerseits durch Hydroxy oder durch geradkettiges oder verzweigtes Alkoxy mit bis zu 4 Kohlenstoffatomen substituiert sein kann,
worin
- R¹ und R²: unter Einbezug der sie verbindenden Doppelbindung gemeinsam einen Phenyl- oder Pyridylring oder einen Ring der Formel 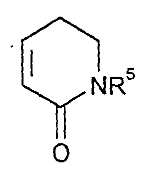 bilden,
worin
R⁵ Wasserstoff oder geradkettiges oder verzweigtes Alkyl mit bis zu 4 Kohlenstoffatomen bedeutet,
- R³ und R⁴: unter Einbezug der sie verbindenden Doppelbindung gemeinsam einen Phenylring oder einen 4- bis 8-gliedrigen Cycloalken- oder Oxocycloalken-Rest bilden,
wobei alle unter R¹/R² und R³/R⁴ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,
- T: für Cycloalkyl mit 4 bis 12 Kohlenstoffatomen oder für geradkettiges oder verzweigtes Alkyl mit bis zu 12 Kohlenstoffatomen steht,
- Q: für Hydroxy oder für einen aktivierenden Rest steht,
und deren Salze erhält,
indem man Verbindungen der allgemeinen Formel (II), 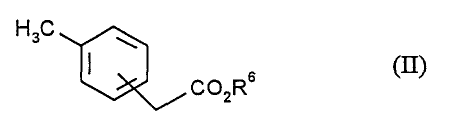 in welcher
- R⁶: zusammen mit dem Sauerstoffatom für einen chiralen Alkoholrest steht, zunächst mit Verbindungen der allgemeinen Formel (III).

T-Z (III)

in welcher
T die angegebene Bedeutung hat und
Z für eine typische Abgangsgruppe wie beispielsweise Brom, Chlor, Iod, Mesyl, Tosyl oder Trifluormethylsulfonyl, vorzugsweise für Iod und Brom steht,
in inerten Lösemitteln in Anwesenheit einer Base durch diastereoselektive Alkylierung in die enantiomerenreinen Verbindungen der allgemeinen Formel (IV) 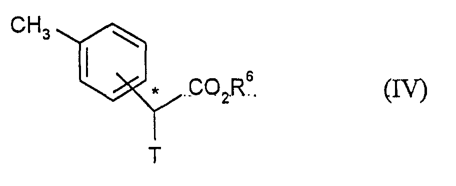 in welcher
- T und R⁶: die angegebene Bedeutung haben,
überführt,
anschließend diese durch Halogenierung in die enantiomerenreinen Verbindungen der allgemeinen Formel (V) 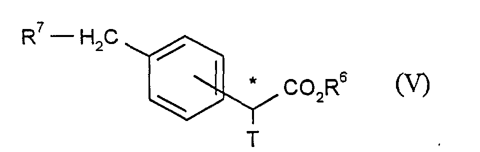 in welcher
- T und R⁶: die angegebene Bedeutung haben
und
- R⁷: für Halogen, wie beispielsweise Chlor, Brom, Jod, vorzugsweise für Brom steht,
überführt,
diese in einem weiteren Schritt durch Umsetzung mit Verbindungen der allgemeinen Formel (VI)

A-H (VI)

in welcher
- R¹, R², R³ und R⁴: die angegebene Bedeutung haben,
die enantiomerenreinen Verbindungen der allgemeinen Formel (VII) 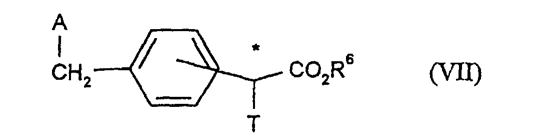 in welcher
- A, T und R⁶: die angegebene Bedeutung haben,
herstellt,
und im Fall der Verbindungen der allgemeinen Formel (I) mit Q = OH eine Hydrolyse durchführt und im Fall Q = aktivierender Rest ausgehend von den enantiomeren reinen Säuren mit aktivierenden Reagenzien umsetzt.

Diese können in einem weiteren Schritt mit D- oder L-Phenylglycinol zu Verbindungen der allgemeinen Formel (VIII) 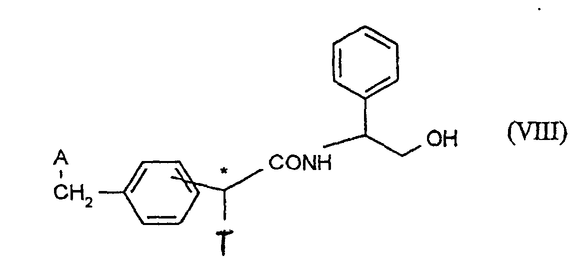 umgesetzt werden,
wobei es sich hierbei um Wirkstoffe für Arzneimittel handelt.

Das erfindungsgemäße Verfahren kann durch folgendes Formelschema beispielhaft erläutert werden:

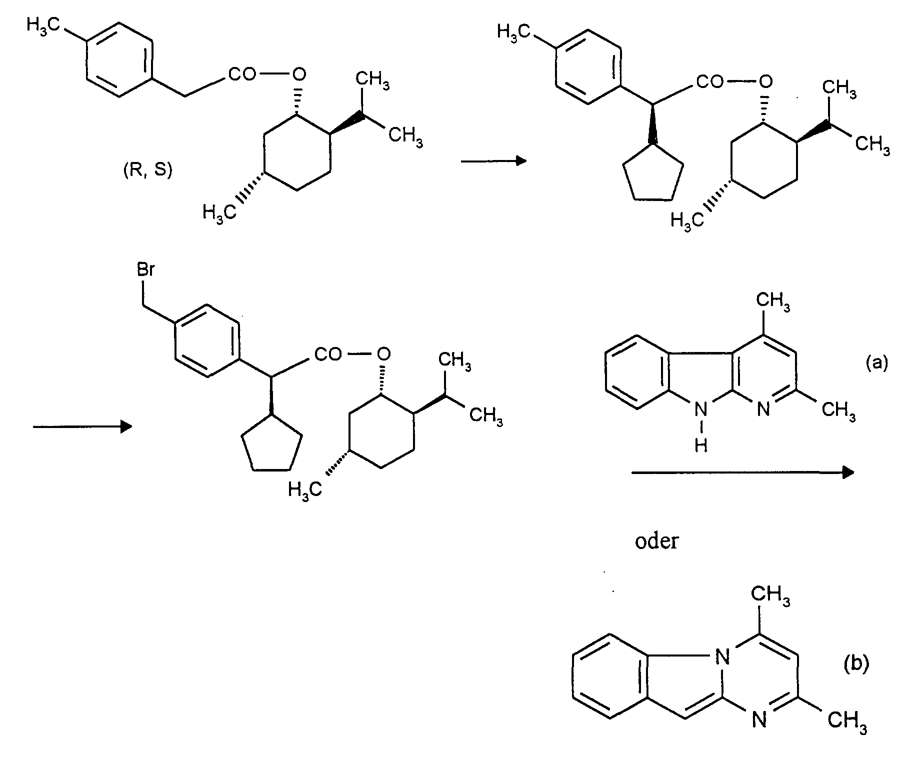

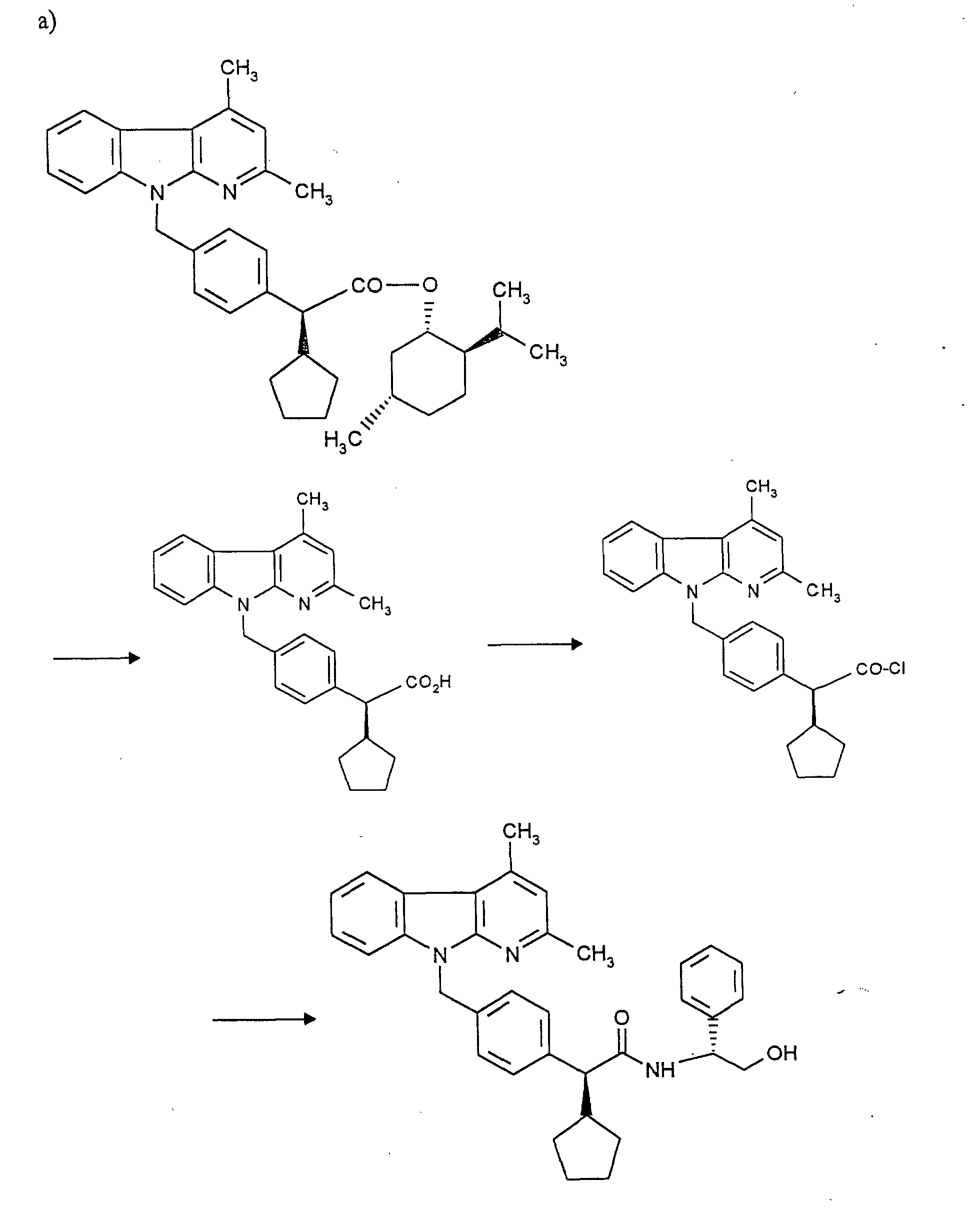

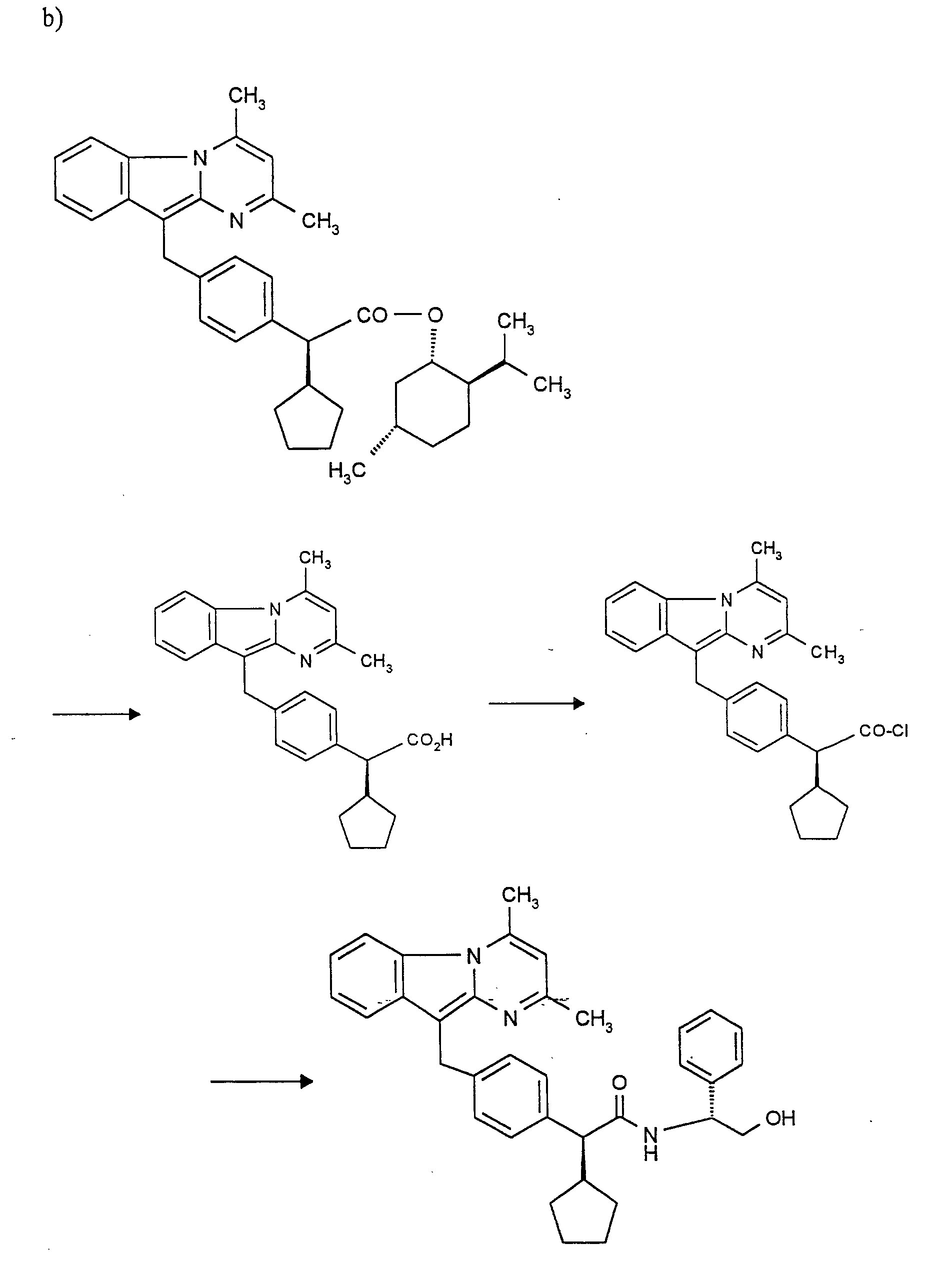

Überraschenderweise liefert das erfindungsgemäße Verfahren die gewünschten enantiomerenreinen Cycloalkano-indol- und azaindol- und Pyrimido-indolcarbonsäuren und deren aktivierte Derivate ohne großen technischen Aufwand in sehr guten Ausbeuten und hoher Reinheit.

Je nach Konfiguration des Restes R⁶ und sterischen Effekten des verwendeten Alkylhalogenids (II) erfolgt die Alkylierung der Verbindung (II) in hohen Ausbeuten und auf einfache Weise erstmals diastereoselektiv. Die Verbindungen (IV) fallen mit hohem Diastereomerenüberschuß an und kristallisieren aus der Reaktionsmischung direkt aus, wodurch bereits die einfache Kristallisation der Rohprodukte die Verbindungen der Formel (IV) in diastereomerenreiner Form liefert.

Ein weiterer Vorteil des erfindungsgemäßen Verfahrens besteht darin, daß durch geeignete Wahl des Lösungsmittels und einer Base das unerwünschte Diastereomer zu dem erwünschten epimerisiert werden kann, welches wiederum direkt auskristallisiert Somit kann aus den Mutterlaugen durch wiederholte Epimerisierung und Kristallisation weiteres (gewünschtes) diasteromerenreines Produkt gewonnen werden. Durch direkte Zumischung der Mutterlaugen in den Alkylierungsschritt kann das gesamte Verfahren in Form eines Kreisprozesses optimiert werden.

Ein weiterer Vorteil des erfindungsgemäßen Verfahrens besteht darin, daß die Umsetzung der halogenierten Verbindungen mit der allgemeinen Formel (V) mit den Verbindungen der allgemeinen Formel (VI) zu den Verbindungen der allgemeinen Formel (VII) überraschenderweise racemisierungsfrei an 2-Kohlenstoffatom zur Carbonsäurefunktion verläuft.

Ein weiterer Vorteil des erfindungsgemäßen Verfahrens ist die am 2-Kohlenstoffatom zur Carbonsäurefunktion racemisierungsfreie Umsetzung der Verbindungen mit der allgemeinen Formel (I) mit Q = aktivierter Rest, vorzugsweise Chlor zu den Verbindungen mit der allgemeinen Formel (VIII).

Weiterhin ist ein großer Vorteil des erfindungsgemäßen Verfahrens, daß die Ausgangsverbindungen sehr gut zugänglich sind. Sie lassen sich aus einfacheren Bausteinen unter geringem technischem Aufwand in guten Ausbeuten herstellen. Darüberhinaus ermöglicht das erfindungsgemäße Verfahren, vorhandene Mengen bekannter Racemate der Verbindungen der allgemeinen Formel (I) in die entsprechenden Enantiomere zu überführen. Das erfindungsgemäße Verfahren ermöglicht die Herstellung der erfindungsgemäßen Verbindungen der allgemeinen Formel (I) mit wenigen Synthesestufen und einer wesentlich höheren Gesamtausbeute als nach aus dem Stand der Technik bekannten Verfahren.

R⁶ steht im Rahmen der oben angegebenen Definition für einen chiralen Alkoholrest wie beispielsweise (+)- oder (-)-Menthyl, (+)- oder (-)-Bornyl, (+)- oder (-)-Isobornyl oder (-)-8-Phenylmenthyl. Bevorzugt steht R⁹ für (+)- oder (-)-Menthyl.

Aktivierende Reste (Q) stehen im Rahmen der Erfindung im allgemeinen für Chlorid, Bromid, Mesylat, Tosylat, Trifluorid. Bevorzugt ist Chlorid.

Bevorzugt werden nach dem erfindungsgemäßen Verfahren Verbindungen der allgemeinen Formel (I) hergestellt, in welchen
- A: für einen Rest der Formel 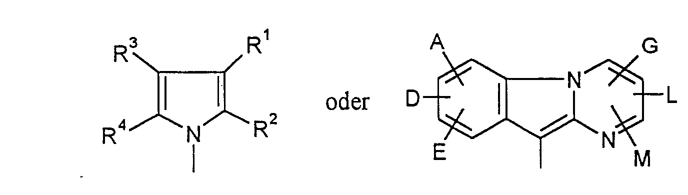 steht,
worin
- A, D, E, G, L und M: gleich oder verschieden sind und Wasserstoff, Fluor, Chlor, Brom, Trifluormethyl, Carboxy, Hydroxy, geradkettiges oder verzweigtes Alkoxy oder Alkoxycarbonyl mit jeweils bis zu 4 Kohlenstoffatomen oder geradkettiges oder verzweigtes Alkyl mit bis zu 4 Kohlenstoffatomen bedeuten, das seinerseits durch Hydroxy oder durch geradkettiges oder verzweigtes Alkoxy mit bis zu 3 Kohlenstoffatomen substituiert sein kann,
- R¹ und R²: unter Einbezug der sie verbindenden Doppelbindung gemeinsam einen Phenyl- oder Pyridylring oder einen Ring der Formel 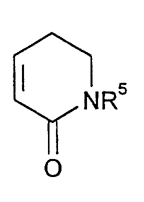 bilden,
worin
R⁵ Wasserstoff oder geradkettiges oder verzweigtes Alkyl mit bis zu 3 Kohlenstoffatomen bedeutet,
- R³ und R⁴: unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter R¹/R² und R³/R⁴ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,
- T: für Cyclobutyl, Cyclopentyl, Cyclohexyl, Cycloheptyl, Cyclooctyl oder für geradkettiges oder verzweigtes Alkyl mit bis zu 10 Kohlenstoffatomen steht,
- Q: für Hydroxy oder für einen aktivierenden Rest steht,
und deren Salze.

Besonders bevorzugt werden nach dem erfindungsgemäßen Verfahren Verbindungen der allgemeinen Formel (I) hergestellt, in welchen
- A: für einen Rest der Formel 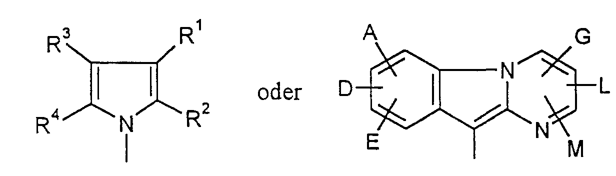 steht,
worin
- A, D, E, G, L und M: gleich oder verschieden sind und Wasserstoff, Fluor, Chlor, Brom, Trifluormethyl, Carboxy, Hydroxy, geradkettiges oder verzweigtes Alkoxy oder Alkoxycarbonyl mit jeweils bis zu 3 Kohlenstoffatomen oder geradkettiges oder verzweigtes Alkyl mit bis zu 3 Kohlenstoffatomen bedeuten,
- R¹ und R²: unter Einbezug der sie verbindenden Doppelbindung gemeinsam einen Phenyl- oder Pyridylring oder einen Ring der Formel 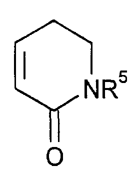 bilden, worin
R⁵ Wasserstoff oder Methyl bedeutet,
- R³ und R⁴: unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter R¹/R² und R³/R⁴ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 4 Kohlenstoffatomen substituiert sind, das seinerseits durch Hydroxy, Methoxy oder Ethoxy substituiert sein kann,
- T: für Cyclopentyl, Cyclohexyl, Cycloheptyl, Cyclooctyl oder für geradkettiges oder verzweigtes Alkyl mit bis zu 6 Kohlenstoffatomen steht,
- Q: für Hydroxy oder für einen aktivierenden Rest steht,
und deren Salze.

Ganz besonders bevorzugt werden die Verbindungen der allgemeinen Formel (I), in welcher
- A: für einen Rest der Formel 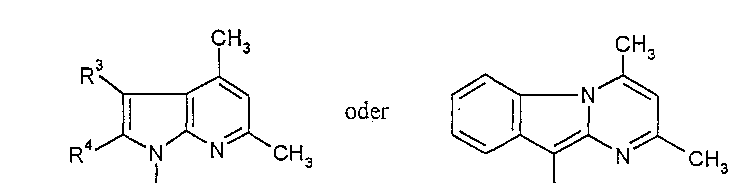 steht,
worin
R³ und R⁴ = Phenylring und mit dem Rest *CH-T-COQ in para-Position und Q = Chlor und deren Salze,
nach dem oben aufgeführten Verfahren hergestellt.

Als Lösemittel für die Alkylierung der Verbindung der allgemeinen Formel (II) eignen sich übliche organische Lösemittel, die sich unter den Reaktionsbedingungen nicht verändern. Hierzu gehören bevorzugt Ether wie Diethylether, Diisopropylether, tert.-Butyl-methylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, N-Methylpyrrolidon, Hexamethylphosphorsäuretriamid, Acetonitril, Aceton oder Nitromethan, Methanol oder Ethanol. Ebenso ist es möglich, Gemische der genannten Lösemittel zu verwenden. Bevorzugt ist Dimethylformamid.

Die Alkylierung wird in den oben aufgeführten Lösemitteln, gegebenenfalls unter Schutzgasatmosphäre, bei Temperaturen von -20°C bis +100°C, vorzugsweise bei -10°C bis +30°C bei Normaldruck durchgeführt.

Als Basen für die diastereoselektive Alkylierung eignen sich die üblichen basischen Verbindungen. Hierzu gehören Alkalihydride wie Natriumhydrid, Alkaliamide wie Natriumamid, Alkalialkoholate wie Natriummethanolat, Natriumethanolat, Kaliummethanolat, Kaliumethanolat oder Kalium-tert.-butylat, oder organische Amine wie Trialkylamine z.B. Triethylamin, oder lithiumorganische Verbindungen wie Butyllithium oder Phenyllithium. Bevorzugt ist Kalium-tert.-butylat.

Bei der diastereoselektiven Alkylierung wird die Base in einer Menge von 1 mol bis 10 mol, bevorzugt von 1,2 mol bis 3 mol bezogen auf 1 mol der Verbindungen der allgemeinen Formel (II) eingesetzt.

Als Lösungsmittel für die Halogenierung der Verbindung der allgemeinen Formel (IV) eignen sich übliche Lösemittel, die sich unter den Reaktionsbedingungen nicht verändern. Hierzu gehören bevorzugt Tetrachlormethan, Chlorbenzol, Dichlorbenzol, Acetonitril, Essigsäure, Schwefelsäure, Nitrobenzol, 1,2-Dichlorethan, Dichlormethan, Trichlormethan.

Für die Halogenierung eignen sich übliche Halogenierungsmittel wie beispielsweise Brom, Chlor, NBS, NCS, Dichlordimethylhydantoin, Dibromdimethylhydantoin,Trichlorisocyanursäure, Chloramin-T.

Als Radikalstarter eignen sich beispielsweise AIBN, Peroxide wie Dibenzoylperoxid, t-Butylhydroperoxid, Dilaurylperoxid, t-Butylperoxid, Perbenzoesäurebutylester, Peroxalsäuredi-t-butylester und photochemische Methoden.

Die Halogenierung wird in den oben aufgeführten Lösemitteln, gegebenenfalls unter Schutzgasatmosphäre, bei Temperaturen von 20°C bis 180°C, gegebenenfalls unter Druck durchgeführt. Vorzugsweise wird die Halogenierung bei 70°C bis 130°C durchgeführt.

Bei der Halogenierung wird das Halogenierungsmittel mit 0,8 mol bis 1,7 mol aktives Halogen, bezogen auf 1 mol der Verbindungen der allgemeinen Formel (I) eingesetzt.

Als Lösemittel für die Alkylierung der Verbindung mit der allgemeinen Formel (VI) eignen sich übliche organische Lösemittel, die sich unter den Reaktionsbedingungen nicht verändern. Hierzu gehören bevorzugt Ether wie Diethylether, Diisopropylether, tert.-Butyl-methylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, N-Methylpyrrolidon, Hexamethylphosphorsäuretriamid, Acetonitril, Aceton oder Nitromethan. Ebenso ist es möglich, Gemische der genannten Lösemittel zu verwenden. Bevorzugt sind Dimethylformamid, Toluol und Tetrahydrofuran.

Die Alkylierung wird in den oben aufgeführten Lösemitteln, gegebenenfalls unter Schutzgasatmosphäre, bei Temperaturen von -20°C bis +100°C, vorzugsweise bei -10°C bis +30°C bei Normaldruck durchgeführt.

Als Basen eignen sich im allgemeinen anorganische oder organische Basen. Hierzu gehören vorzugsweise Alkalihydroxide wie z.B. Natriumhydroxid oder Kaliumhydroxid, Erdalkalihydroxide wie z.B. Bariumhydroxid, Alkalicarbonate und -hydrogencarbonate wie Natriumcarbonat, Natriumhydrogencarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.-butylat, oder organische Amine (Trialkyl(C₁-C₆)amine) wie Triethylamin, oder Heterocyclen wie 1,4-Diazabicyclo[2,2,2]octan (DABCO), 1,8-Diazabicyclo[5,4,0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrogencarbonat, Kaliumcarbonat und Kalium-tert.-butylat, DBU oder DABCO.

Bei der Alkylierung wird die Base in einer Menge von 1 mol bis 10 mol, bevorzugt von 1,2 mol bis 3 mol, bezogen auf 1 mol der Verbindungen der allgemeinen Formel (II) eingesetzt.

Für die Abspaltung des chiralen Restes R⁶ in den Verbindungen der allgemeinen Formel (VII) eignen sich die üblichen organischen Carbonsäuren, wie beispielsweise Essigsäure, Ameisensäure, Trifluoressigsäure, Methansulfonsäure oder anorganische Säuren wie beispielsweise Bromwasserstoffsäure, Chlorwasserstoffsäure oder Schwefelsäure oder Gemische der genannten Säuren. Bevorzugt sind Essigsäure, Ameisensäure, Bromwasserstoffsäure und/oder Schwefelsäure. Ganz besonders bevorzugt ist das Gemisch Essigsäure/Schwefelsäure sowie Ameisensäure/Bromwasserstoffsäure und Ameisensäure/Schwefelsäure.

Die Säuren bzw. deren Gemische werden gleichzeitig als Lösemittel verwandt und somit in einem großen Überschuß eingesetzt.

Die Abspaltung erfolgt in einem Temperaturbereich von 0°C bis +150°C, bevorzugt von 40°C bis 100°C.

Sie kann im allgemeinen bei Normaldruck, gegebenenfalls aber auch bei Überoder Unterdruck (z.B. 0,5 bis 3 bar) durchgeführt werden.

Nach Neutralisation mit Basen in Wasser oder in einem der oben aufgeführten Lösemittel, insbesondere in einem Wasser/Toluol-, Wasser/Isopropanol-, Wasser/ Methanol- und Wasser/Ethanol-Gemisch werden die Säuren nach üblicher Methode aufgearbeitet.

Als Basen zur Neutralisation eignen sich Alkalihydroxide wie Natriumhydroxid oder Kaliumhydroxid. Bevorzugt ist Natriumhydroxid.

Als Lösemittel für die Aktivierung der Verbindungen der allgemeinen Formel (I) eignen sich übliche organische Lösemittel, die sich unter den Reaktionsbedingungen nicht verändern. Hierzu gehören bevorzugt Ether wie Diethylether, Diisopropylether, tert.-Butyl-methylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dimethylformamid, Toluol und Dichlormethan.

Für die Aktivierung eignen sich übliche Aktivierungsmittel wie beispielsweise Oxalylchlorid, Phosphortrichlorid, Phosphorpentachlorid, Trichlorisocyanosäure, Thionylchlorid, Phosphortribromid, Phosphorpentabromid, Mesylchlorid, Tosylchlorid, Phosgen, Trifluormethansulfonsäurechlorid, Sulfurylchlorid. Bevorzugt werden Thionylchlorid und Oxalylchlorid und Phosgen

Die Aktivierung wird in den oben aufgeführten Lösemitteln, gegebenenfalls unter Schutzgasatmosphäre bei Temperaturen von -20°C bis 120°C, gegebenenfalls unter Druck durchgeführt. Vorzugsweise wird die Aktivierung bei -20°C bis 80°C durchgeführt.

Bei der Aktivierung wird das Aktivierungsreagenz mit 1 mol bis 10 mol bezogen auf 1 mol der Verbindung der allgemeinen Formel (I) eingesetzt oder gegebenenfalls als Lösungsmittel verwendet.

Die Aktivierung erfolgt gegebenenfalls unter Zusatz von Basen wie organische Amine (Trialkyl(C₁-C₆)amine) wie Triethylamin, oder Heterocyclen wie 1,4-Diazabicyclo[2,2,2]octan (DABCO), 1,8-Diazabicyclo[5,4,0]undec-7-en (DBU), Pyridin, Diaminopyridin, Methylpiperidin oder Morpholin. Gegebenenfalls können die aktivierten Derivate ausgehend von Carbonsäuresalzen der Alkali- und Erdalkalimetalle durch Umsetzung mit z.B. Oxalylchlorid hergestellt werden.

Die Verbindungen der allgemeinen Formel (II), 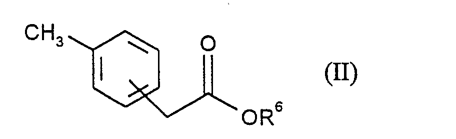 in welcher
- R⁶: für einen chiralen Alkoholrest steht,
werden erhalten,
indem man Verbindungen der allgemeinen Formel (IX) 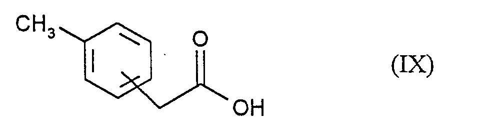 mit chiralen Alkoholen nach literaturbekannten Verfahren verestert.

Die Verbindungen der allgemeinen Formel (IX) sind an sich bekannt oder nach üblichen Methoden herstellbar.

Die enantiomerenreinen Verbindungen der allgemeinen Formel (I), in welcher Q für tert.-Butoxy steht, sind neu und können hergestellt werden, indem man zunächst racemische Carbonsäuren der allgemeinen Formel (X) 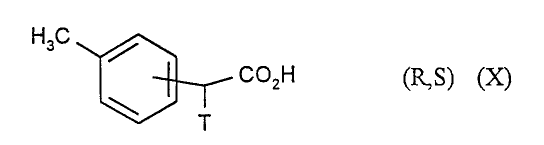 in welcher
- T: die oben angegebene Bedeutung hat,
durch Umsetzung mit (R)- oder (S)-Phenylethylamin in inerten Lösemitteln und anschließender Kristallisation der Phenethylammoniumsalze und anschließende Hydrolyse der Salze in die enantiomerenreinen Verbindungen der allgemeinen Formel (XI) 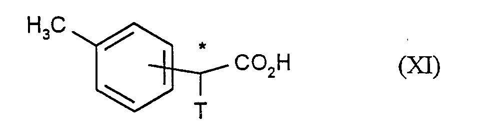 in welcher
- T: die oben angegebene Bedeutung hat,
überführt,
diese in einem weiteren Schritt mit Isobuten, in inerten Lösemitteln und in Anwesenheit von Säuren in die enantiomerenreinen Ester (XII) überführt 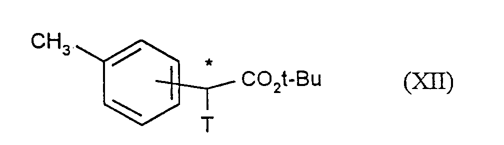 in welcher
- T: die oben angegebene Bedeutung hat,
anschließend werden die Ester (XII) durch Halogenierung in die enantiomerenreinen Verbindungen der allgemeinen Formel (XIII) 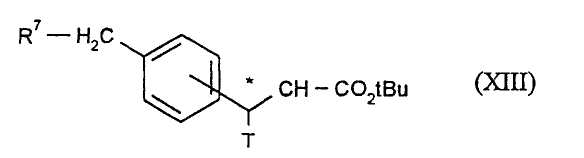 in welcher
- T: die oben angegebene Bedeutung hat
und
- R⁷: für eine typische Abgangsgruppe wie beispielsweise Chlor, Brom, Jod, Tosylat oder Mesylat, vorzugsweise für Brom steht,
überführt,
in einem weiteren Schritt durch Umsetzung mit Verbindungen der allgemeinen Formel (VI)

A-H (VI)

in welcher
- A: die oben angegebene Bedeutung hat,
die enantiomerenreinen Verbindungen der allgemeinen Formel (I) 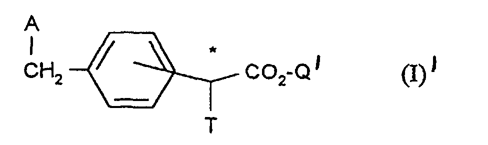 in welcher
- A und T: die oben angegebene Bedeutung haben und
- Q': für tert.-Butyl steht,
herstellt,
und im Fall der Verbindungen der allgemeinen Formel (I) mit Q = OH eine Hydrolyse durchführt.

Die Verseifung von tert.-Butylestern erfolgt im allgemeinen mit Sauren, wie beispielsweise Salzsäure oder Trifluoresssigsäure, in Anwesenheit eines der oben angegebenen Lösemitteln und/oder Wasser oder deren Gemische, vorzugsweise mit Dioxan oder Tetrahydrofuran.

Die Verbindungen der allgemeinen Formel (X) werden aus den entsprechenden literaturbekannten Estern durch Hydrolyse nach literaturbekannten Methoden hergestellt.

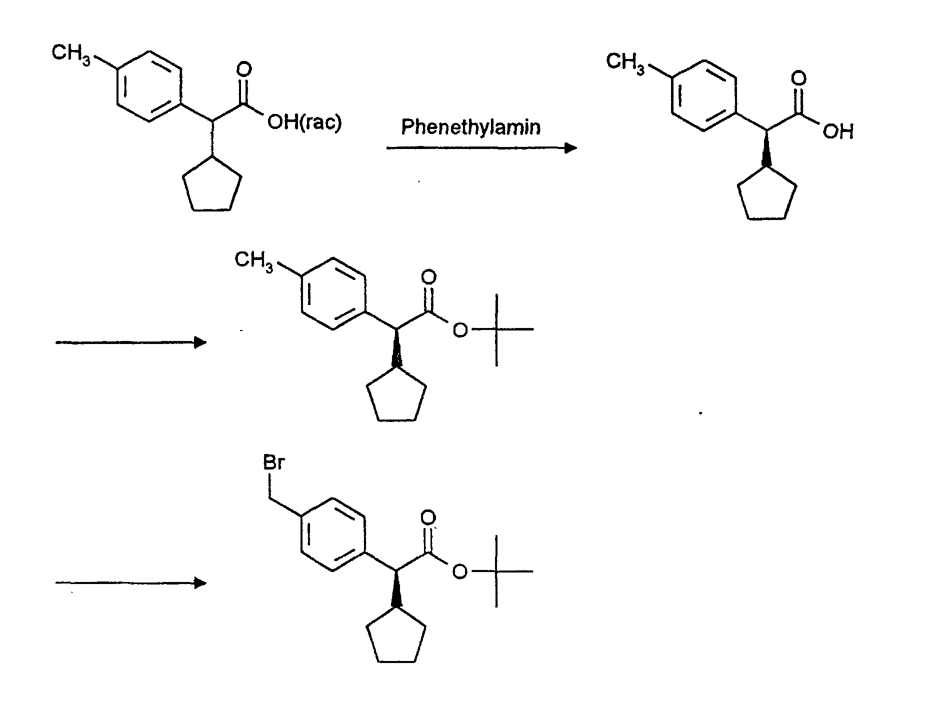

### Beispiel I

### 2(R/S)-2-Cyclopentyl-2-(4-methylphenyl)-essigsäure

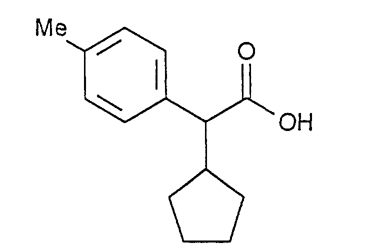

In einem 40 l-Rührwerkkessel mit angeschlossenem Waschturm werden 2,0 kg (7,2 mol) 2(R,S)-2-Cyclopentyl-2-(4-methylphenyl)-essigsäure-tert.-butylester in 4 l Dioxan gelöst. Nach Zugabe von 4,5 l konzentrierter Salzsäure wird bis zum vollständigen Umsatz (3h) bei 50°C gerührt. Das Reaktionsgemisch wird mit Eis versetzt und mit konzentrierter Natronlauge auf pH = 12 eingestellt. Nach Zugabe von Wasser bis zur vollständigen Auflösung der Feststoffe wird mit Essigsäure gewaschen, die organische Phase wird mit verdünnter Natronlauge gewaschen und die vereinigten wäßrigen Phasen unter Kühlung mit konzentrierter Salzsäure auf pH = 1 eingestellt. Es wird zweimal mit Essigester gewaschen, über Natriumsulfat getrocknet und eingeengt.
Ausbeute: 1,27 kg; 81% d.Th.
Schmpkt.: 92°C
R_{f}= 0,20 (Petrolether: Essigester = 4:1)
¹H-NMR (CDCl₃, 200 MHz, TMS): δ = 0,98 (m, 1H); 1,20- 1,71 (m, 6H); 1,82 - 2,05 (m, 1H); 2,31 (s, 3H); 2,52 (m, 1H); 3,21 (d, 1H); 7,10 (m, 2H); 7,21 m, 2H); 11,90 (br, s, 1H) ppm.

### Beispiel II

### (S)-2-Cyclopentyl-2-(4-methylphenyl)-essigsäure

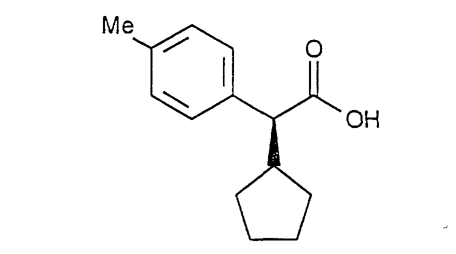

Zu einer Suspension von 560 g (2,57 mol) der Verbindung aus Beispiel I in 4,8 l Wasser werden unter Rühren 2,4 l THF und 129,7 g (1,28 mol) Triethylamin gegeben. Die entstehende Lösung wird auf 60°C erwärmt, es werden 155,4 g (1,28 mmol) (S)-(-)-Phenethylamin zugegeben und die anfallende Suspension 2 h bei 60°C gerührt. Das Reaktionsgemisch wird auf 20°C gekühlt, der Niederschlag wird abgesaugt, mit 2,4 l Wasser / THF (2:1) gewaschen und im Vakuum getrocknet.
Ausbeute: 360 g Phenethylammoniumsalz; 41,3% d.Th.
In 3 l Wasser werden 745 g (2,2 mol) Phenethylammoniumsalz suspendiert, mit verdünnter Salzsäure (1:1) angesäuert (pH = 1) und 30 Minuten gerührt. Die ölige Suspension wird dreimal mit je 1 l Dichlormethan gewaschen, die vereinigten organischen Phasen mit Wasser gewaschen, über Natriumsulfat getrocknet und eingeengt, wobei der Rückstand kristallisiert.
Ausbeute: 475 g; 37,3% d.Th. bezogen auf Racemat Bsp.-Nr. I
ee: 96,3% (HPLC)
Schmpkt.: 66°C

Durch Kristallisation des Phenethylammoniumsalzes aus THF, wie oben beschrieben wird. das Reinenantiomer erhalten:
ee: >99,5% (HPLC)
Drehwert: [α]²⁰_{D} = +59,55 (Ethanol / c = 0,85)
Die HPLC-Methode zur Bestimmugn des ee.Wertes ist folgende:

| | |
|---|---|
| Säule | Chiracel OJ (Daicel) |
| Partikelgröße | 10 µ |
| Packing | 250 x 2 mm (Fa. Grom) |
| Mobile Phase | n-Heptan: 2-Propanol = 97:3 |
| Fluß | 0,2 ml/min |
| Eingangsdruck | 22 bar |

### Beispiel III

### (S)-2-Cyclopentyl-2-(4-methylphenyl)essigsäure-tert.butylester

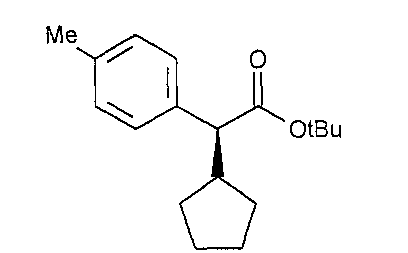

Zu einer Lösung von 465 g (2,13 mol) der Verbindung aus Beispiel II in 1,4 l Dichlormethan werden 6 ml konzentrierte Schwefelsäure gegeben, wobei sich eine Temperatur von ca. 10°C einstellt. In ein Dewargefäß werden 550 ml (5 mol) Isobuten einkondensiert und in einer Portion zur Eduktlösung gegeben. Das Reaktionsgemisch wird über Nacht gerührt. Zur Vervollständigung des Umsatzes werden nochmals 6 ml konzentrierte Schwefelsäure und 500 ml Isobuten zugegeben und über Nacht gerührt. Nach Zugabe von 40 g Kaliumcarbonat wird 3 h gerührt, und darauf 2 l Wasser zugegeben, wobei es anfangs zu einer starken Gasentwicklung kommt. Es wird dreimal mit je 2 l Dichlormethan gewaschen, die vereinigten organischen Phasen werden mit 5 l Natriumchloridlösung gewaschen, über Natriumsulfat getrocknet und zu einem Öl, das langsam durchkristallisiert, eingeengt.
Ausbeute: 480 g; 82% d.Th.
Schmpkt.: 45°C
R_{f} = 0,90 (Toluol : Essigester = 8:2)

### Beispiel IV

### (S)-2-(4-Brommethylphenyl)-2-cyclopentyl-essigsäure-tert.butylester

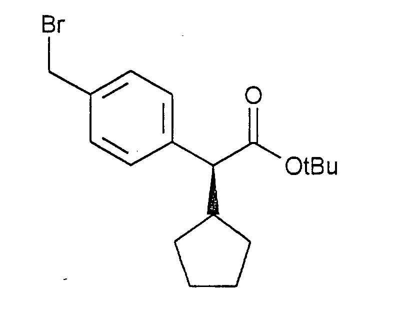

In einem 10 l-Kolben werden 480 g (1,75 mol) der Verbindung aus Beispiel III in 3,4 l Tetrachlormethan unter Rückfluß gelöst und mit 70 g Gesamtmenge von 311 g (1,75 mol) NBS sowie 14 g (0,085 mol) AIBN versetzt. Die Reaktion setzt nach ca 1 h Refluxieren ein; nach Abklingen wird weiteres NBS in 50 g Portionen zugegeben. Nach 5 h Refluxieren und anschließendem Stehen bei Raumtemperatur über Nacht wird zur Aufarbeitung auf 0°C gekühlt, das Succinimid abgesaugt und mit 600 ml Tetrachlormethan nachgewaschen. Die vereinigten Filtrate werden eingeengt und Restlösemittel bis zur Gewichtskonstanz im Vakuum entfernt.
Rohausbeute: 570 g; ca. 100% d.Th.
HPLC: 68,8% (15,5% Edukt, 10,1% Dibromverbindung)

Der Reinstoff wird durch Säulenchromatographie erhalten
R_{f} = 0,42 (Petrolether, Essigester = 20/1)
¹H-NMR (CDCl₃, 200 MHz, TMS): δ = 0,98 (m, 1H); 1,22 - 1,71 (m, 6H); 1,40 (s, 9H); 1,90 (m, 1H); 2,47 (m, 1H); 3,16 (d, 1H); 4,49 (s, 2H); 7,32 (m, 4H) ppm.

### Beispiel V

### 2-(4-Tolyl)-essigsäure-(L)-menthylester

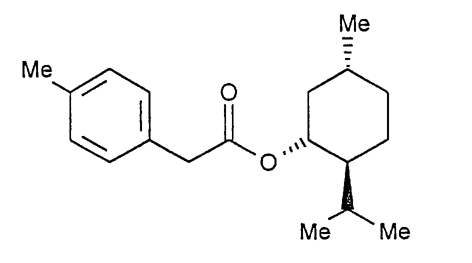

3,15 kg p-Tolylessigsäure und 9,45 l Toluol werden vorgelegt. Unter Rühren und Kühlen werden 3,115 kg L-Menthol und 21,4 ml Methansulfonsäure zugegeben. Anschließend wird auf Rückflußtemperatur erhitzt und innerhalb von 16 bis 20 Stunden über einen Wasserabscheider die entsprechende Menge Wasser abgetrennt. Nach Abkühlen auf Raumtemperatur wird einmal mit 4,41 l gesättigter Natriumhydrogencarbonat-Lösung und zweimal mit je 4,41 l Wasser ausgerührt. Die organische Phase wird vom Lösemittel befreit und ergibt 5,725 kg gewünschte Verbindung (GC 99,9%, Retentionszeit 19,49 min).
¹H-NMR (CDCl₃, ppm): 7,05 - 7,15 (4H, m); 4,55 (1H, txd); 3,5 (2H, s); 2,8 (3H, s)); 0,65 (3H, s).

### Beispiel VI

### 2-(S)-2-Cyclopentyl-2-(4-tolyl)-essigsäure-(L)-menthylester

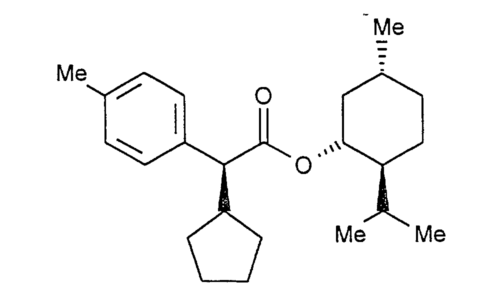

1,575 kg Kalium-tert.butanolat werden bei Raumtemperatur in 3,75 l DMF gelöst. Man kühlt auf 10°C ab und läßt innerhalb von 45 Minuten bei dieser Temperatur 2,678 kg der Verbindung aus Beispiel V zulaufen und spült mit 0,375 l DMF nach. Innerhalb von 1 bis 2 Stunden werden nun unter voller Kühlung 1,658 kg Cyclopentylbromid zugepumpt. Die Suspension wird ohne Kühlung noch eine Stunde nachgerührt und dann auf -7°C abgekühlt. Bei Erreichen von -10°C wird mit dem richtigen Diastereomeren angeimpft und dann weiter auf -7°C gekühlt. Nach Erreichen von -7°C wird 3 bis 4 Stunden bei dieser Temperatur nachgerührt. Die Aufarbeitung erfolgt durch Einbringen der Reaktionssuspension in ein Gemisch aus 1,5 kg Eis und 6 kg Wasser. Der Ansatz wird danach bei 0 bis 2°C über Nacht gerührt. Die Aufarbeitung erfolgt durch Absaugen der Suspension und Waschen der Kristalle mit insgesamt 2,5 l Wasser. Die Kristalle werden bei 45°C im Vakuumtrockenschrank getrocknet. Es werden 3,289 kg eines 85 zu 15 Diastereomerengemisches erhalten.

4,345 kg einer wie oben beschriebenen, hergestellten Mischung, werden in 21,75 l bei 30 bis 35°C gelöst. Nach Animpfen mit dem richtigen Diastereomeren und Abkühlen auf Raumtemperatur wird über Nacht gerührt und am nächsten Morgen auf 0 bis 5°C gekühlt. Nach 1 bis 2 Stunden bei dieser Temperatur werden die Kristalle abgesaugt, getrocknet oder erneut umkristallisiert. Durch ein bis zweimaliges Wiederholen der Methanolkristallisation kann Material mit einer Diastereomerenreinheit von ≥99,5% hergestellt werden (GC-Retentionszeit 22,61 min).

Die Ausbeute an diastereomerenreiner Titelverbindung liegt bei 65-70% über die Stufen Cyclopentylierung und Reinkristallisation und kann durch Rekristallisation bzw. durch Epimerisierung der Mutterlaugen mit Kalium-tert.butanolat in DMF und erneute Kristallisation des rohen Diastereomerengemisches auf 75-80% gesteigert werden.
¹³C-NMR (CDCl₃, CH-Signale, ppm) 128,90; 128,92; 73,96; 57,85; 46,92; 43,13; 31,28; 25,96.

### Beispiel VII

### 2-(S)-2-(4-Brommethyl-phenyl)-2-cyclopentyl-essigsäure-(L)-menthylester

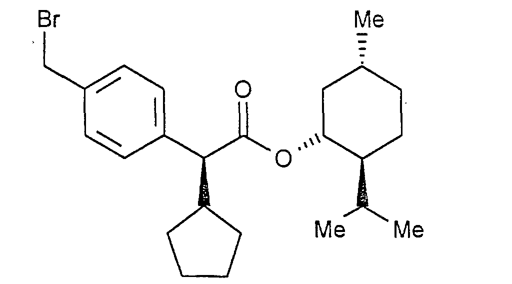

1,40 kg der Verbindung aus Beispiel VI werden in 13,74 l Chlorbenzol auf 80°C erwärmt. Danach gibt man 0,618 kg 1,3-Dibrom-5,5-dimethylhydantoin hinzu und erwärmt weiter auf 85°C. Bei dieser Temperatur wird nun zum Starten der Reaktion 20,4 g AIBN gegeben. Die Temperatur steigt nach Beginn der Reaktion auf 90 bis 105°C, sinkt dann aber wieder auf etwa 85°C ab. Insgesamt wird 2 Stunden nachreagiert. Danach wird der Kesselinhalt auf Raumtemperatur abgekühlt und eine Stunde nachgerührt. Es wird von den ausgefallenen Kristallen abgesaugt und das Filtrat vom Lösemittel befreit. Das zurückbleibende Öl ist nach HPLC-Analyse (Retentionszeit: 14,68 min) 61,2%ig. Es werden 1,69 kg erhalten. Das Gemisch kann roh in die folgenden Alkylierungen eingesetzt werden. Chromatographie und nachfolgende Kristallisation liefern ein weißes Pulver vom Schmelzpunkt 57-58°C, mit korrekter CH-Analyse.
¹H-NMR (CDCl₃, ppm): 7,3 (4H, s); 4,65 (1H, txd); 4,45 (2H, s); 3,35 (1H, d); 0,65 (3H, d).

### Beispiel VIII

### 2(S)-2-Cyclopentyl-2[4-(2,4-dimethyl-α-carbolin-9-yl)methyl]phenyl-essigsäure-Lmenthylester

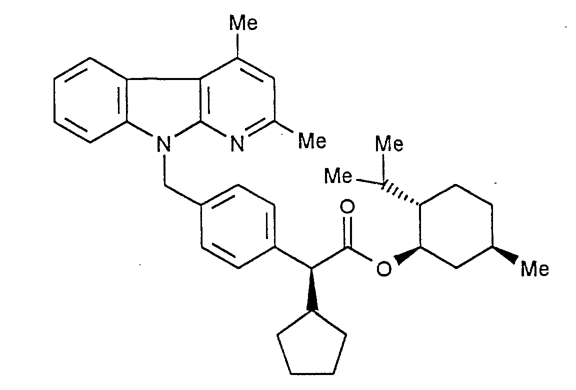

Die Reaktion wird unter Stickstoffatmosphäre durchgeführt. 480 g (2,44 mol) Carbolin werden in 4,13 l Dimethylformamid suspendiert und unter Rühren mit 287,7 g Kalium-tert.butylat gelöst in 1 l Dimethylformamid versetzt. Die Reaktionslösung erwärmt sich auf 30°C. Nach 30 min wird der Ansatz auf 20°C abgekühlt. Anschließend werden 1,707 kg (2,69 mol) 69%iges Menthylesterbromid, gelöst in 1,56 l Dimethylformamid, so zugetropft, daß die Innentemperatur nicht über 35°C ansteigt. Nach weiteren 15 min Reaktionszeit wird die Reaktionslösung in ein Gemisch aus 1,8 l 10 %ige Natriumchloridlösung und 13 l Essigester gegossen. Nach 20 min unter Rühren wird die Essigesterphase abgetrennt und zweimal mit je 3 l 10%iger Natriumchloridlösung extrahiert. Nach dem Trocknen der organischen Phase über Natriumsulfat wird Essigester im Vakuum bei ca. 40°C abdestilliert. Der sirupöse Rückstand wird in 4,4 l Methanol aufgenommen und 30 min unter Rückfluß 12 h bei Raumtemperatur gerührt. Die ausgefallenen Kristalle werden abgesaugt, mit Methanol gewaschen und im Vakuum bei 40°C getrocknet.
Ausbeute: 947 g (70,6% d.Th.)
Schmelzpunkt: 142°C

### Beispiel IX

### 2-(S)-2-Cyclopentyl-2-[4-(2,4-dimethyl-α-carbolin-9-yl)-methyl]phenylessigsäure

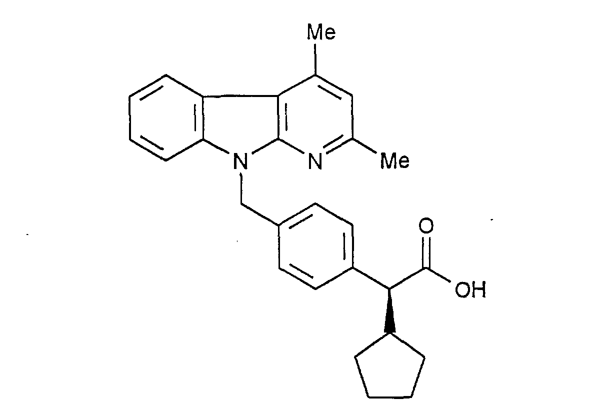

947 g (1,72 mol) der Verbindung aus Beispiel VIII werden mit 2,4 l Ameisensäure versetzt. Unter Rühren werden 1,21 l wäßrige Bromwasserstoffsäure (48%ig) zugetropft. Die erhaltene Suspension wird 6 Stunden bei 95-98°C gerührt und anschließend auf Raumtemperatur abgekühlt. Die Reaktionslösung wird unter Rühren mit 1,6 l Isopropanol und 3,2 l Wasser versetzt. Unter leichter Kühlung wird mit 45%iger Natronlauge ein pH-Wert von 5 eingestellt (Verbrauch an Natronlauge: 5,2 kg). Der Niederschlag wird abgesaugt, zweimal mit 5,7 l Wasser gewaschen und trocken gesaugt. Anschließend wird das wasserfeuchte Produkt in 2,6 l Isopropanol 2 Stunden bei Raumtemperatur ausgerührt. Das Kristallisat wird abgesaugt, mit 2,8 l Isopropanol nachgewaschen und im Vakuum bei 60°C getrocknet.
Ausbeute: 574 g (81% d.Th.)
Schmelzpunkt: 197-199°C

### Beispiel X

### 2-(S)-2-Cyclopentyl-2-[4-(2,4-dimethyl-α-carbolin-9-yl)-methyl]phenylessigsäurechlorid

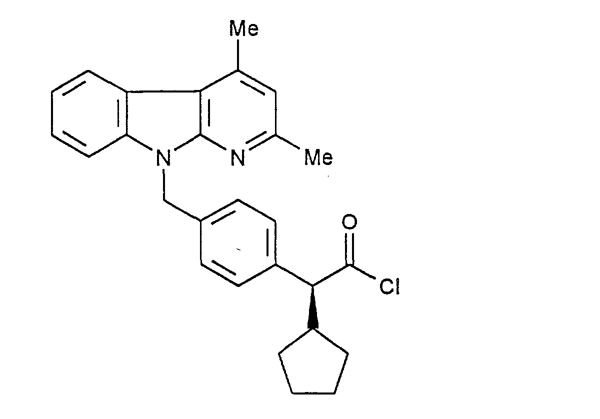

Eine Suspension von 350 g (0,85 mol) der Verbindung aus Beispiel IX in 3 l Methylenchlorid wird unter Rühren zum Rückfluß erhitzt. Innerhalb von 1 h werden 95 ml (155 g, 1,3 mol) Thionylchlorid zugetropft und weitere 2 h unter Rückflußtemperatur gerührt. Anschließend wird die Reaktionslösung auf Raumtemperatur abgekühlt, bei 25-30°C im Vakuum bis zur beginnenden Kristallisation eingeengt und mit 2,5 l Toluol versetzt. Bei einer Temperatur von 30-40°C werden im Vakuum weitere 2,3 l Lösemittel abdestilliert. Nach Abkühlen auf ca. 20°C werden 1,2 l Toluol zu dem Ansatz gegeben. Die Suspension wird auf 0-5°C abgekühlt, 1 h bei dieser Temperatur gerührt, abgesaugt und mit 1,4 l Toluol nachgewaschen und trocken gesaugt. Das toluolfeuchte Produkt wird ohne weitere Charakterisierung umgesetzt.

### Beispiel XI

### 2-(S)-2-Cyclopentyl-2-[4-(2,4-dimethyl-α-carbolin-9-yl)-methyl]phenylessigsäure-[(R)phenylglycinol]amid

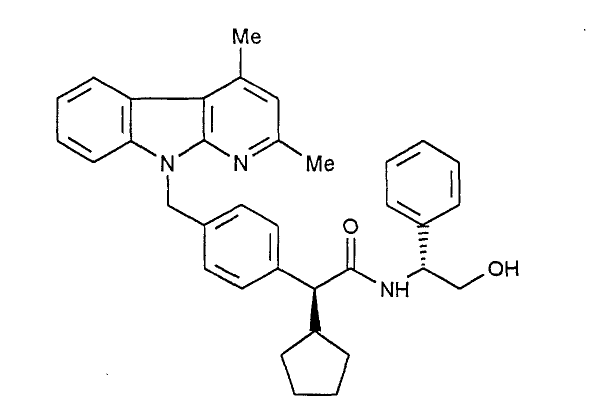

458 g toluolfeuchtes Säurechlorid, 125 g R-Phenylglycinol und 8,5 Ltr. Toluol werden ineinem 20 l Planschliffkolben eingebracht und gerührt. Beginnend bei 20°C werden innerhalb von 15 min 235 ml (171 g, 1,7 mol) Triethylamin zugetropft. Anschließend wird 1 Stunde bei 60-65°C gerührt, auf RT abgekühlt und 8 h bei dieser Temperatur gerührt. Die ausgefallenen Kristalle werden abgesaugt, mit Toluol nachgewaschen und trockengesaugt. Nachdem die toluolfeuchten Kristalle 15 min in 11 Ltr. Ethanol zum Sieden erhitzt wurden, werden 7,5 Ltr. Ethanol abdestilliert und anschließend 8 Ltr. Wasser in der Siedehitze zugegeben. Es werden weitere 15 min bei Rückflußtemperatur gerührt. Der Kolbeninhalt wird auf 20°C abgekühlt. Die Kristalle werden abgesaugt, dreimal mit jeweils 3,5 Ltr. Wasser gewaschen und bei 80°C im Vakuum getrocknet. Das getrocknete Rohprodukt aus ca. 4 Ltr. Methylethylketon umkristallisiert.
Ausbeute: 383 g (85% d. Th.)
Schmelzpunkt: 221°C

### Beispiel XII

### 2-(S)-2-Cyclopentyl-2-[4-(2,4-dimethyl-pyrimido[1,2-a]-indol-9-yl)-methyl)phenyl]-essigsäure-L-menthylester

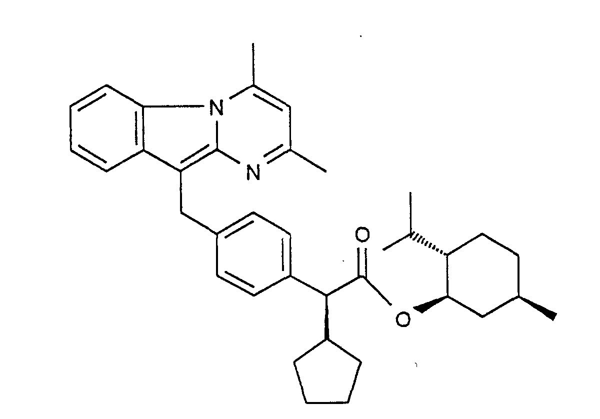

41,9 g (0,2 mol) 2,4-Dimethyl-pyrimido[1,2-a]indol und 33,6 g Natriumhydrogencarbonat werden in 300 ml Dimethylformamid vorgelegt. Das Gemisch wird auf 120°C erwärmt und innerhalb 10 min bei 30 bis 70°C eine Lösung von 128,1 g (0,2 mol, 68 %ig) der Verbindung aus Beispiel XII (Bromid) in 135 ml Dimethylformamid zugetropft. Es wird 40 min bei 120°C gerührt und das Reaktionsgemisch bei Raumtemperatur auf 2,2 l halbkonzentrierte Natriumchloridlösung gegossen. Nach Extraktion mit 2,2 l Essigester wird die organische Phase dreimal mit halbkonzentrierter Natriumchloridlösung gewaschen, über Natriumsulfat getrocknet und bei 50°C eingeengt.
Ausbeute: 165,4 g (70,4 % d. Th.)
HPLC: 46,9 %

### Beispiel XIII

### 2-(S)-2-Cyclopentyl-2-[4-(2,4-dimethyl-pyrimido[1,2-a]-indol-9-yl)-methyl)phenyl]-essigsäure-menthyl-ester-hydrochlorid

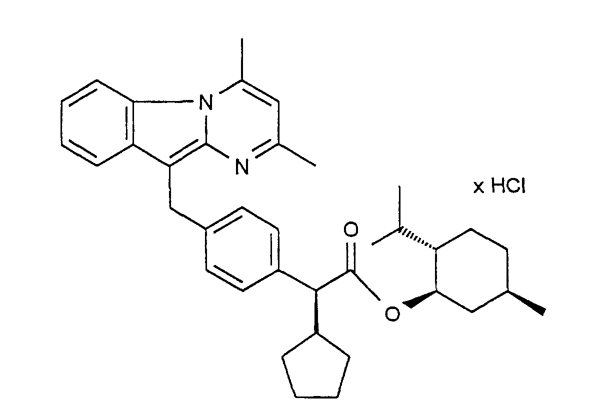

165,4 g (0,14 mol) des Rohprodukts aus Beispiel XII werden bei 50°C in 1,6 l Aceton gelöst. Innerhalb 10 min werden bei 15 bis 20°C 80 ml (0,48 mol) halbkonzentrierte Salzsäure zugetropft und 2 h bei ca. 10°C gerührt. Der ausgefallene Feststoff wird abgesaugt, mit wenig Aceton gewaschen und bei 50°C im Vakuum getrocknet.
Ausbeute: 60,7 g (39,3 % d. Th., bezogen auf Pyrimidoindol)
HPLC: 76,1 %

### Beispiel XIV

### 2-(S)-2-Cyclopentyl-2-[4-(2,4-dimethyl-pyrimido[1,2-a]-indol-9-yl)-methyl)phenyl]-essigsäure

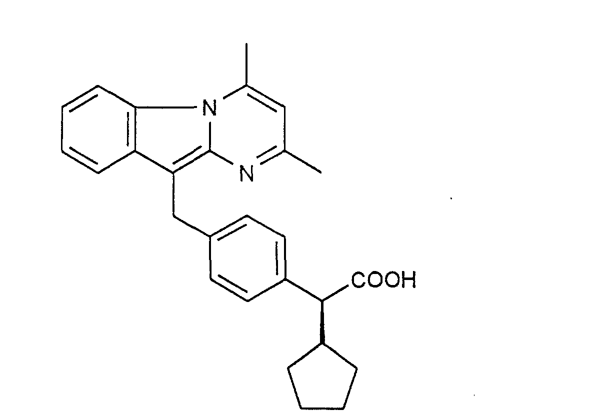

60,7 g (0,10 mol 76,1 %ig) der Verbindung aus Beispiel XII werden in 146 ml Ameisensäure und 43 ml 48 %iger Bromwasserstoffsäure gelöst und 6 h unter Rückfluß (109°C) gerührt, wobei das Reaktionsgemisch anfangs stark aufschäumt. Bei Raumtemperatur werden 94 ml Isopropanol und 187 ml Wasser zugegeben und unter Kühlung innerhalb 1 h durch Zugabe von 190 ml konzentrierter Natronlauge pH 5 eingestellt. Es wird 2 h gerührt, der Feststoff wird abgesaugt und dreimal mit je 100 ml Isopropanol und dreimal mit je 100 ml Wasser gewaschen. Der Rückstand wird 1 h mit 310 ml Isopropanol verrührt, abgesaugt, mit wenig Isopropanol gewaschen und im Vakuum bei 50°C getrocknet.
Ausbeute: 36,9 g (ca. 100 % d. Th.)
HPLC: 92,1 %

### Beispiel XV

### 2-(S)-2-Cyclopentyl-2-[4-(2,4-dimethyl-pyrimido[1,2-a]-indol-9-yl)-methyl)phenyl]-essigsäure-chlorid

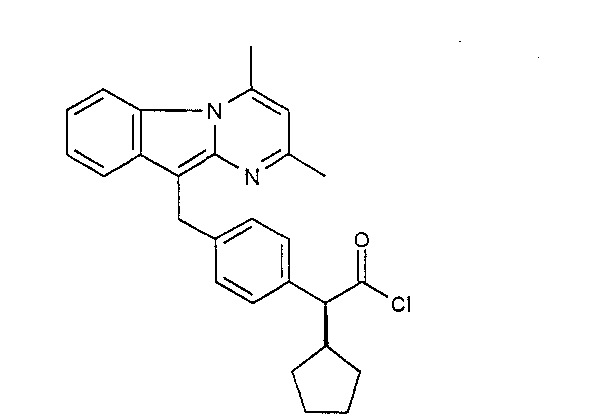

Zu einer Lösung von 37,1 g (0,09 mol) der Verbindung aus Beispiel XIV in 306 ml Dichlormethan werden bei 39°C innerhalb 10 min 10 ml (0,14 mol) Thionylchlorid getropft, die entstehenden Gase werden in einem Waschturm geleitet. Es wird 2 h unter Rückfluß gerührt und flüchtige Anteile bei 40°C Badtemperatur im Vakuum abdestilliert. Die verbleibende dicke Suspension wird mit 270 ml Toluol versetzt, bei 50°C im Vakuum eingeengt und der Rückstand bei Raumtemperatur 2 h mit 270 ml Toluol verrührt. Das Produkt wird abgesaugt, mit wenig Toluol gewaschen und im Vakuum getrocknet.
Ausbeute: 47 g (toluolfeucht)

### Beispiel XVI

### 2-(S)-2-Cyclopentyl-2-[4-(2,4-dimethyl-pyrimido[1,2-a]-indol-9-yl)-methyl)phenyl]-essigsäure[(R)phenylglycinol]amid

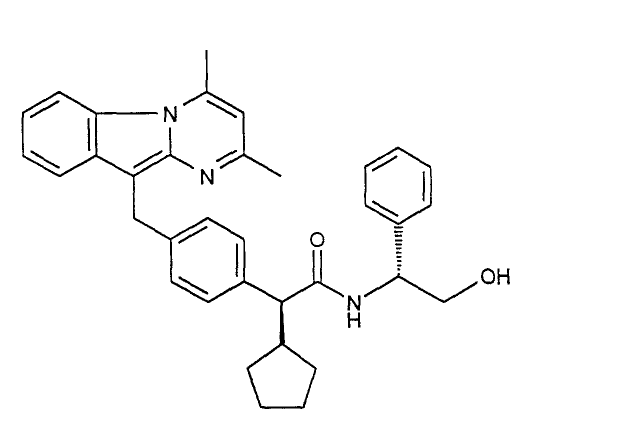

Das toluolfeuchte Rohprodukt (47 g, ca. 0,08 mol) aus Beispiel XV wird in 810 ml Toluol suspendiert. Es werden 11,8 g (0,086 mol) D-Phenylglycinol und 23 ml (0,166 mol) Triethylamin zugegeben und 1 h bei 61 bis 63°C gerührt. Bei Raumtemperatur wird der Feststoff abgesaugt und mit 500 ml Wasser und 50 ml gesättigter Natriumhydrogencarbonatlösung 2 h gerührt. Der Feststoff wird abgesaugt, mit 150 ml Wasser gewaschen und im Vakuum bei 50°C getrocknet.

Das Rohprodukt (32,3 g) wird in 1 l Methylethylketon in der Siedehitze gelöst, heiß von unlöslichen Teilen abgesaugt, das Filtrat wird auf ca. 200 ml aufkonzentriert und mit einem Eisbad gekühlt. Das auskristallisierte Produkt wird abgesaugt, im Vakuum bei 50°C getrocknet, in 2 l Methanol in der Siedehitze gelöst, heiß abgesaugt und auf 150 ml aufkonzentriert. Das bei Raumtemperatur ausgefallene Produkt wird abgesaugt, mit 150 ml Methanol gewaschen und im Vakuum bei 50°C getrocknet.
Ausbeute: 14,9 g (34,6 % d. Th.)
HPLC: 99,9 %
Schmelzpunkt: 195-200°C

## Patentansprüche

1. Verfahren zur Herstellung von enantiomerenreinen Cycloalkano-indol- und azaindol-carbonsäuren und Pyrimido[1,2a]indolcarbonsäuren und deren aktivierten Derivaten der allgemeinen Formel (I) 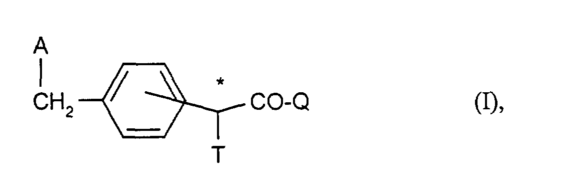 in welcher
A für einen Rest der Formel 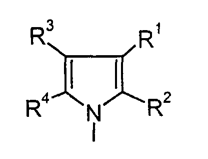 oder 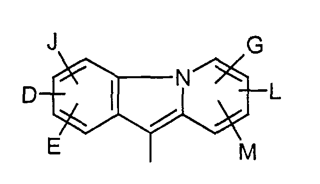 steht,
J, D, E, G, L und M gleich oder verschieden sind und Wasserstoff, Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen bedeuten, das seinerseits durch Hydroxy oder durch geradkettiges oder verzweigtes Alkoxy mit bis zu 4 Kohlenstoffatomen substituiert sein kann,
worin
R¹ und R² unter Einbezug der sie verbindenden Doppelbindung gemeinsam einen Phenyl- oder Pyridylring oder einen Ring der Formel 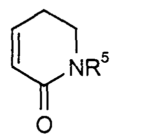 bilden,
worin
R⁵ Wasserstoff oder geradkettiges oder verzweigtes Alkyl mit bis zu 4 Kohlenstoffatomen bedeutet,
R³ und R⁴ unter Einbezug der sie verbindenden Doppelbindung gemeinsam einen Phenylring oder einen 4- bis 8-gliedrigen Cycloalken- oder Oxocycloalken-Rest bilden,
wobei alle unter R¹/R² und R³/R⁴ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,
T für Cycloalkyl mit 4 bis 12 Kohlenstoffatomen oder für geradkettiges oder verzweigtes Alkyl mit bis zu 12 Kohlenstoffatomen steht,
Q für Hydroxy oder für einen aktivierenden Rest, ausgewählt aus der Gruppe Chlorid, Bromid, Mesylat, Tosylat, Triflat, steht,
und deren Salze,
indem man Verbindungen der allgemeinen Formel (II), 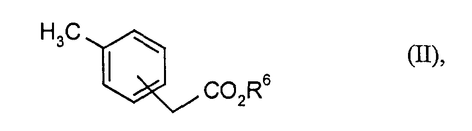 in welcher
R⁶ zusammen mit dem Sauerstoffatom für einen chiralen Alkoholrest, ausgewählt aus der Gruppe (+)- oder (-)-Menthyl, (+)- oder (-)-Bornyl, (+)- oder (-)-Isobornyl oder (-)-8-Phenylmenthyl, steht, zunächst mit Verbindungen der allgemeinen Formel (III)
T-Z (III)
in welcher
T die angegebene Bedeutung hat und
Z für eine typische Abgangsgruppe wie beispielsweise Brom, Chlor, Iod, Mesyl, Tosyl oder Trifluormethylsulfonyl, vorzugsweise für Iod und Brom steht,
in inerten organischen Lösemitteln in Anwesenheit einer Base durch diastereoselektive Alkylierung in die enantiomerenreinen Verbindungen der allgemeinen Formel (IV) 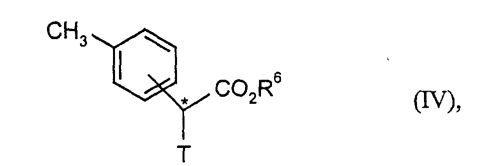 in welcher
T und R⁶ die angegebene Bedeutung haben,
überführt,
anschließend diese durch Halogenierung in die enantiomerenreinen Verbindungen der allgemeinen Formel (V) 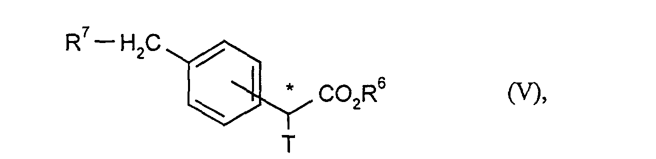 in welcher
T und R⁶ die angegebene Bedeutung haben
und
R⁷ für Halogen, wie beispielsweise Chlor, Brom, Jod, vorzugsweise für Brom steht,
überführt,
diese in einem weiteren Schritt durch Umsetzung mit Verbindungen der allgemeinen Formel (VI)
A-H (VI)
in welcher
A die angegebene Bedeutung hat,
die enantiomerenreinen Verbindungen der allgemeinen Formel (VII) 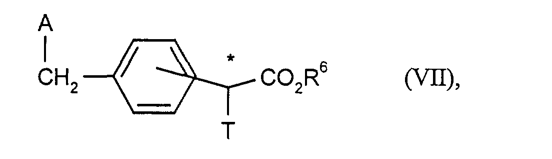 in welcher
A, T und R⁶ die angegebene Bedeutung haben,
herstellt,
und im Fall der Verbindungen der allgemeinen Formel (I) mit Q = OH eine Hydrolyse durchführt und im Fall Q = aktivierender Rest ausgehend von den enantiomeren reinen Säuren mit aktivierenden Reagenzien umsetzt.

2. Verfahren nach Anspruch 1 zur Herstellung von Verbindungen der Formel (I) nach Anspruch 1, worin
R¹ und R² unter Einbezug der sie verbindenden Doppelbindung gemeinsam einen Phenyl- oder Pyridylring oder einen Ring der Formel 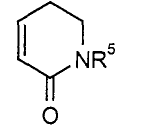 bilden,
worin
R⁵ Wasserstoff oder geradkettiges oder verzweigtes Alkyl mit bis zu 3 Kohlenstoffatomen bedeutet,
R³ und R⁴ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter R¹/R² und R³/R⁴ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,
T für Cyclobutyl, Cyclopentyl, Cyclohexyl, Cycloheptyl, Cyclooctyl oder für geradkettiges oder verzweigtes Alkyl mit bis zu 10 Kohlenstoffatomen steht,
Q für Hydroxy oder für einen aktivierenden Rest, ausgewählt aus der Gruppe Chlorid, Bromid, Mesylat, Tosylat, Triflat, steht,
und deren Salze.

3. Verfahren nach Anspruch 1 zur Herstellung von Verbindungen der Formel (I) nach Anspruch 1
worin
R¹ und R² unter Einbezug der sie verbindenden Doppelbindung gemeinsam einen
Phenyl- oder Pyridylring oder einen Ring der Formel 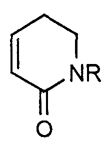 bilden,
worin
R⁵ Wasserstoff oder Methyl bedeutet,
R³ und R⁴ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden.

4. Verfahren nach Anspruch 1 zur Herstellung von Verbindungen der allgemeinen Formel (I) nach Anspruch 1,
worin
A für einen Rest der Formel 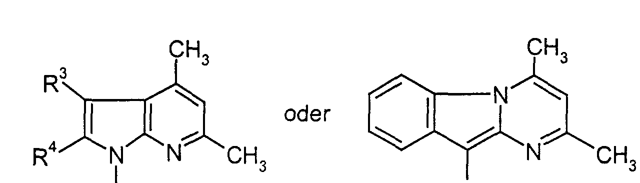 steht,
und
R³ und R⁴ = Phenylring
und mit dem Rest *CH-T-COQ in para-Position und Q = Chlor bedeuten.

5. Verfahren gemäß einem der Ansprüche 1 bis 4, bei dem die Alkylierung von Verbindungen der Formel (II) mit Verbindungen der Formel (III) zu Verbindungen der Formel (IV) in Dimethylformamid mit Kalium-tert.-butanolat als Base durchgeführt wird.

6. Verfahren zur Herstellung von 2-(S)-2-Cyclopentyl-2-[4-(2,4-dimethyl-α-carbolin-9-yl)-methyl]phenylessigsäure der Formel 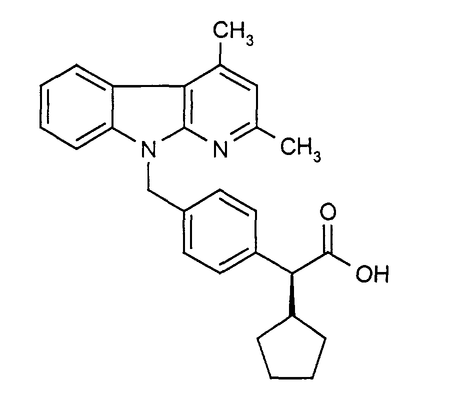 indem man
2-(4-Tolyl)-essigsäure-(L)-menthylester der Formel 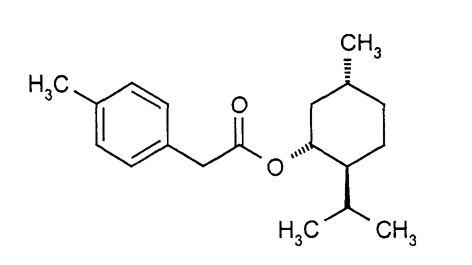 mit Cyclopentylbromid in Dimethylformamid in Anwesenheit von Kaliumtert.-butanolat durch diastereoselektive Alkylierung in 2-(S)-2-Cyclopentyl-2-(4-tolyl)-essigsäure-(L)-menthylester der Formel 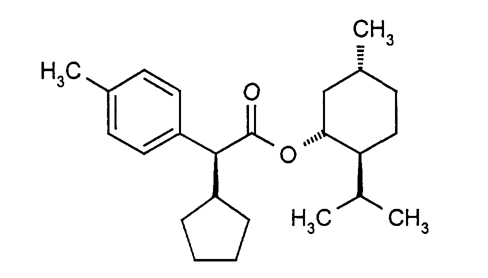 überführt,
anschließend diese Verbindung durch Halogenierung in 2-(S)-2-(4-Brommethyl-phenyl)-2-cyclopentyl-essigsäure-(L)-menthylester der Formel 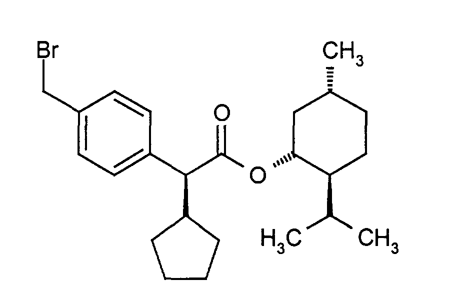 überführt,
aus dieser Verbindung einem weiteren Schritt durch Umsetzung mit α-Carbolin der Formel 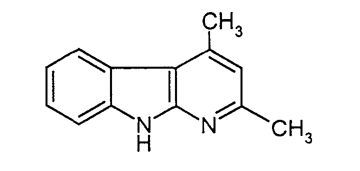 2-(S)-2-Cyclopentyl-2-[4-(2,4-dimethyl-α-carbolin-9-yl)-methyl]-phenylessigsäure-(L)-menthylester der Formel 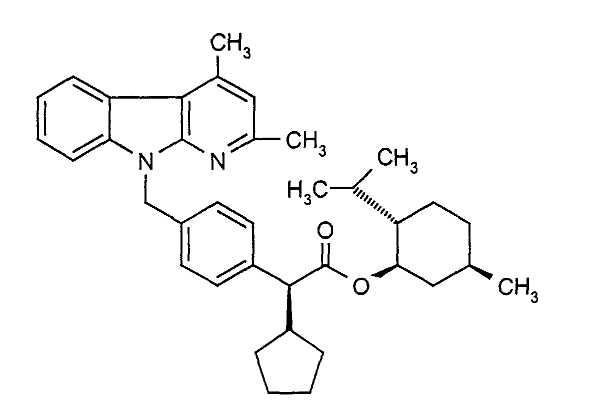 herstellt und diese Verbindung hydrolysiert.

7. Verfahren zur Herstellung von enantiomerenreinen Cycloalkanoindol- und azaindol- und Pyrimido[1,2a]indolcarbonsäuren und deren Derivaten der allgemeinen Formel (I) 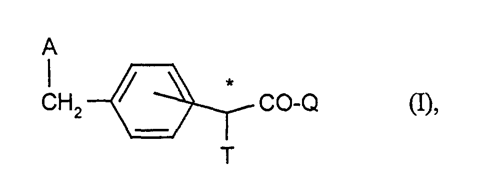 in welcher
A für einen Rest der Formel 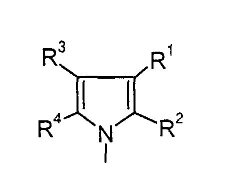
oder 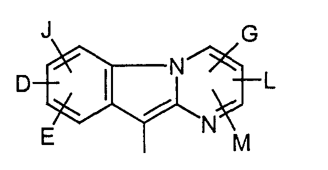 steht,
J, D, E, G, L und M gleich oder verschieden sind und Wasserstoff, Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen bedeuten, das seinerseits durch Hydroxy oder durch geradkettiges oder verzweigtes Alkoxy mit bis zu 4 Kohlenstoffatomen substituiert sein kann,
worin
R¹ und R² unter Einbezug der sie verbindenden Doppelbindung gemeinsam einen Phenyl- oder Pyridylring oder einen Ring der Formel 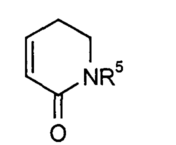 bilden,
worin
R⁵ Wasserstoff oder geradkettiges oder verzweigtes Alkyl mit bis zu 4 Kohlenstoffatomen bedeutet,
R³ und R⁴ unter Einbezug der sie verbindenden Doppelbindung gemeinsam einen Phenylring oder einen 4- bis 8-gliedrigen Cycloalken- oder Oxocycloalken-Rest bilden,
wobei alle unter R¹/R² und R³/R⁴ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,
T für Cycloalkyl mit 4 bis 12 Kohlenstoffatomen oder für geradkettiges oder verzweigtes Alkyl mit bis zu 12 Kohlenstoffatomen steht,
Q für Hydroxy oder für einen aktivierenden Rest, ausgewählt aus der Gruppe Chlorid, Bromid, Mesylat, Tosylat, Triflat, steht,
und deren Salze erhält,
**dadurch gekennzeichnet, daß** man zunächst racemische Carbonsäuren der allgemeinen Formel (X) 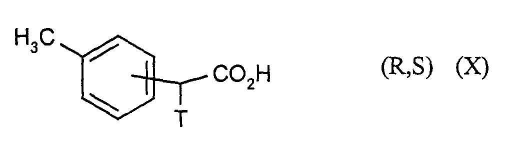 in welcher
T die oben angegebene Bedeutung hat,
durch Umsetzung mit (R)- oder (S)-Phenylethylamin in inerten Lösemitteln und anschließender Kristallisation der Phenethylammoniumsalze und anschließende Hydrolyse der Salze in die enantiomerenreinen Verbindungen der allgemeinen Formel (XI) 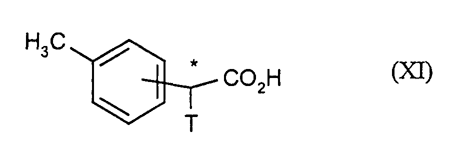 in welcher
T die oben angegebene Bedeutung hat,
überführt,
diese in einem weiteren Schritt mit Isobuten, in inerten Lösemitteln und in Anwesenheit von Säuren in die enantiomerenreinen Ester (XII) 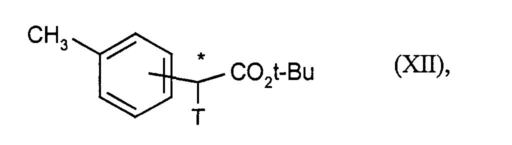 in welcher
T die oben angegebene Bedeutung hat,
überführt,
anschließend werden die Ester (XII) durch Halogenierung in die enantiomerenreinen Verbindungen der allgemeinen Formel (XIII) 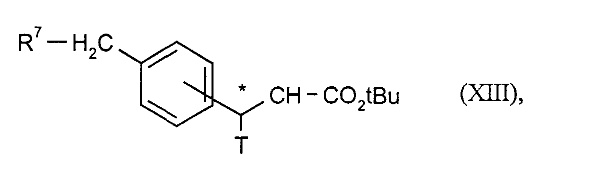 in welcher
R die oben angegebene Bedeutung hat
und
R⁷ für eine typische Abgangsgruppe wie beispielsweise Chlor, Brom, Jod, Tosylat oder Mesylat, steht,
überführt,
in einem weiteren Schritt durch Umsetzung mit Verbindungen der allgemeinen Formel (VI)
A-H (VI)
in welcher
A die oben angegebene Bedeutung hat,
die enantiomerenreinen Verbindungen der allgemeinen Formel (I) 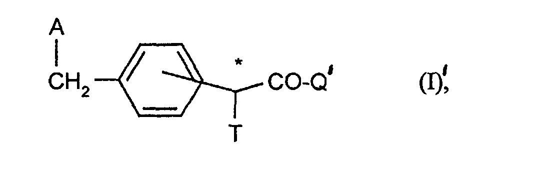 in welcher
A und T die oben angegebene Bedeutung haben und
Q' für tert.-Butyl steht,
herstellt,
und im Fall der Verbindungen der allgemeinen Formel (I) mit Q = OH eine Hydrolyse durchführt.

8. Zwischenprodukte der allgemeinen Formel 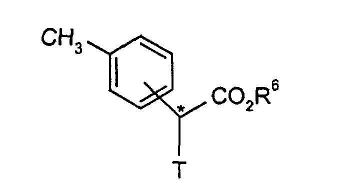 in welcher
T für Cycloalkyl mit 4 bis 12 Kohlenstoffatomen oder für geradkettiges oder verzweigtes Alkyl mit bis zu 12 Kohlenstoffatomen steht
und
R⁶ für den D- oder L-Menthylrest oder tert.-Butyl steht,
wobei die Verbindung mit T = Isopropyl ausgenommen ist.

9. Zwischenprodukte der allgemeinen Formel 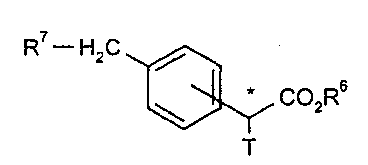 in welcher
R⁶ für den D- oder L-Menthylrest oder für den tert.-Butylrest steht,
T für Cycloalkyl mit 4 bis 12 Kohlenstoffatomen oder für geradkettiges oder verzweigtes Alkyl mit bis zu 12 Kohlenstoffatomen steht
und
R⁷ für Brom steht.

10. Verwendung der Zwischenprodukte nach Anspruch 8 und 9 zur Herstellung von enantiomerenreinen Wirkstoffen der Formel (I) nach Anspruch 1,
worin
Q für einen Phenylglycinolrest steht.

11. Zwischenprodukte der allgemeinen Formel (I) 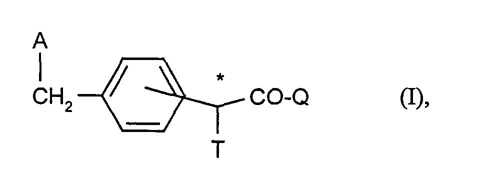 in welcher A für 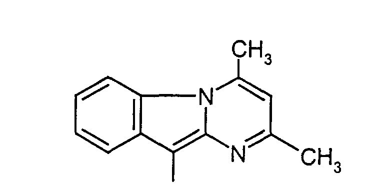 und T für Cycloalkyl mit 4-12 Kohlenstoffatomen oder für geradkettiges oder verzweigtes Alkyl mit bis zu 12 Kohlenstoffatomen steht und
Q für Hydroxy oder Chlor steht und deren Salze.

12. Zwischenprodukte der allgemeinen Formel (VII) 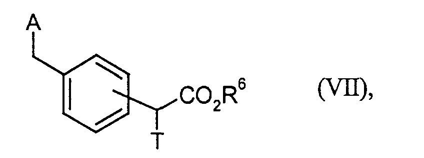 in welcher A für 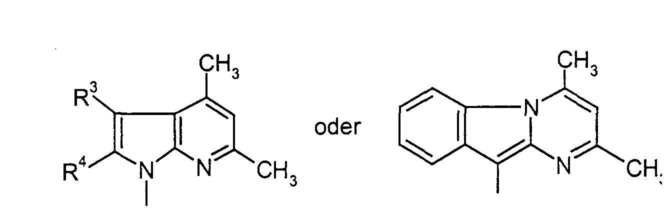 steht,
R³ und R⁴ die oben angegebene Bedeutung haben,
T für Cycloalkyl mit 4 bis 12 Kohlenstoffatomen oder für geradkettiges oder verzweigtes Alkyl mit bis zu 12 Kohlenstoffatomen steht und
R⁶ für D- oder L-Menthyl steht.

13. Zwischenverbindungen der Reihe:
a) (S)-2-(4-Brommethylphenyl)-2-cyclopentyl-essigsäure-tert.butylester der Formel 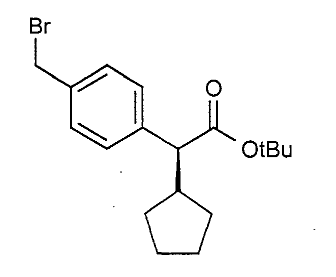 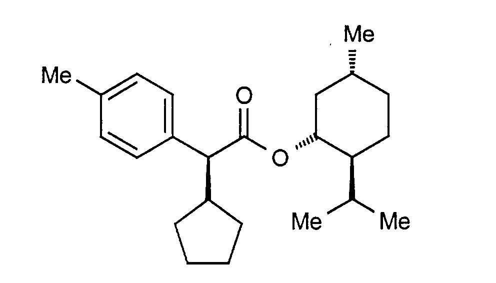
c) 2-(S)-2-(4-Brommethyl-phenyl)-2-cyclopentyl-essigsäure-(L)-menthylester der Formel 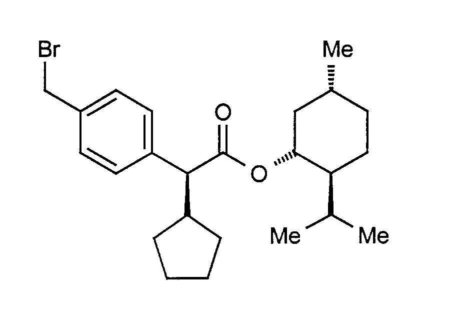
d) 2(S)-2-Cyclopentyl-2[4-(2,4-dimethyl-α-carbolin-9-yl)methyl]phenylessigsäure-L-menthylester der Formel 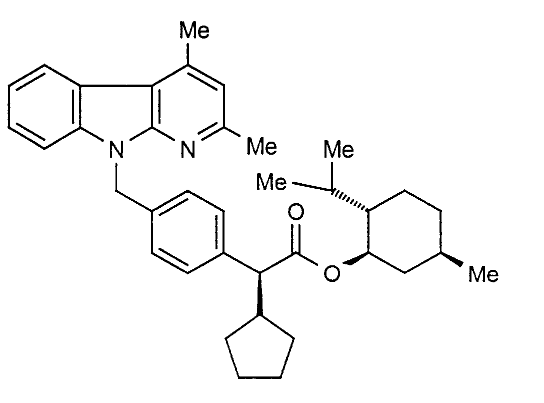
e) 2-(S)-2-Cyclopentyl-2-[4-(2,4-dimethyl-α-carbolin-9-yl)-methyl]phenylessigsäure-chlorid der Formel 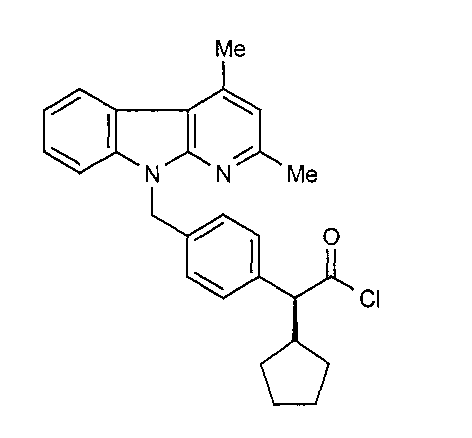
f) 2-(S)-2-Cyclopentyl-2-[4-(2,4-dimethyl-pyrimido[1,2-a]-indol-9-yl)-methyl)phenyl]-essigsäure-L-menthylester der Formel 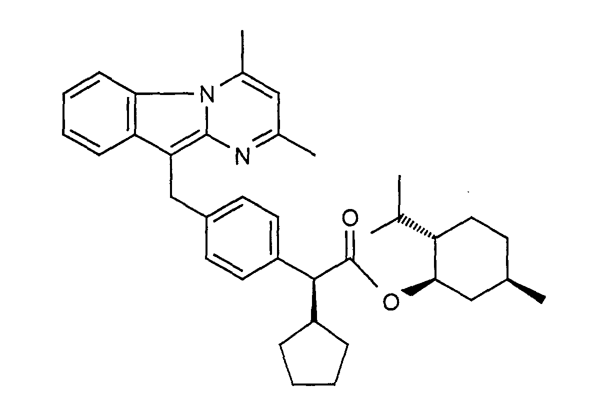
g) 2-(S)-2-Cyclopentyl-2-[4-(2,4-dimethyl-pyrimido[1,2-a]-indol-9-yl)-methyl)phenyl]-essigsäure-menthyl-ester-hydrochlorid der Formel 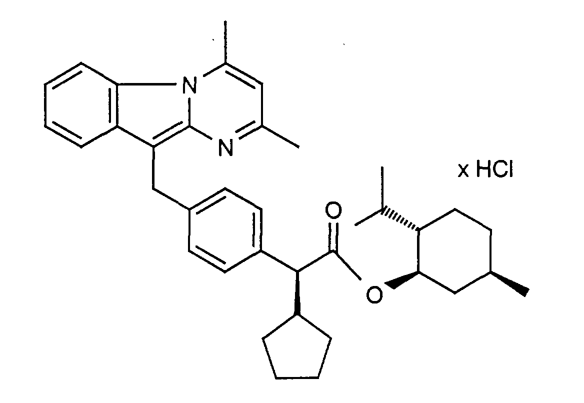
h) 2-(S)-2-Cyclopentyl-2-[4-(2,4-dimethyl-pyrimido[1,2-a]-indol-9-yl)-methyl)phenyl]-essigsäure-chlorid der Formel

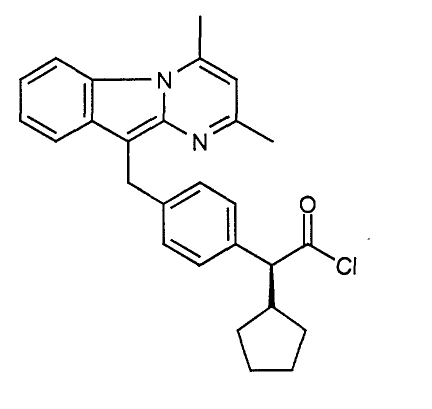

## Claims

1. Process for the preparation of enantiomerically pure cycloalkano-indole-carboxylic acids and azaindole-carboxylic acids and pyrimido[1,2a]-indolecarboxylic acids and their activated derivatives of the general formula (I) 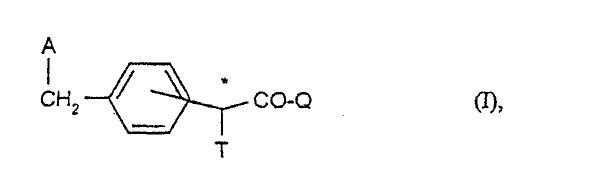 in which
A represents a radical of the formula 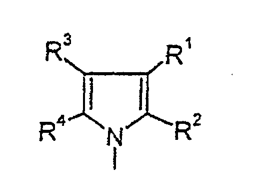 or 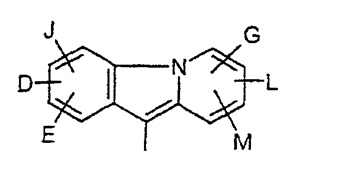
J, D, E, G, L and M are identical or different and denote hydrogen, halogen, trifluoromethyl, carboxyl, hydroxyl, linear or branched alkoxy or alkoxycarbonyl each having up to 6 carbon atoms or linear or branched alkyl having up to 6 carbon atoms, which itself can be substituted by hydroxyl or by linear or branched alkoxy having up to 4 carbon atoms,
in which
R¹ and R², including the double bond linking them, together form a phenyl ring or pyridyl ring or a ring of the formula 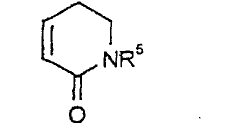 where
R⁵ denotes hydrogen or linear or branched alkyl having up to 4 carbon atoms,
R³ and R⁴, including the double bond linking them, together form a phenyl ring or a 4- to 8-membered cycloalkene or oxocycloalkene radical,
where all the ring systems listed under R¹/R² and R³/R⁴ are optionally up to trisubstituted identically or differently by halogen, trifluoromethyl, carboxyl, hydroxyl, by linear or branched alkoxy or alkoxycarbonyl each having up to 6 carbon atoms, or by linear or branched alkyl having up to 6 carbon atoms, which itself can be substituted by hydroxyl or by linear or branched alkoxy having up to 4 carbon atoms,
T represents cycloalkyl having 4 to 12 carbon atoms, or represents linear or branched alkyl having up to 12 carbon atoms,
Q represents hydroxyl or an activating radical selected from the group consisting of chloride, bromide, mesylate, tosylate and triflate,
and their salts,
by firstly converting compounds of the general formula (II), 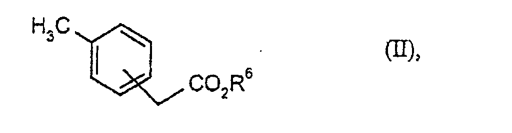 in which
R⁶ together with the oxygen atom represents a chiral alcohol radical selected from the group consisting of (+)- or (-)-menthyl, (+)- or (-)-bornyl, (+)- or (-)-isobornyl or (-)-8-phenylmenthyl, by means of compounds of the general formula (III)
T-Z (III)
in which
T has the meaning specified and
Z represents a typical leaving group, such as bromine, chlorine, iodine, mesyl, tosyl, or trifluoromethylsulphonyl, preferably iodine or bromine,
in inert organic solvents in the presence of a base by diastereoselective alkylation into the enantiomerically pure compounds of the general formula (IV) 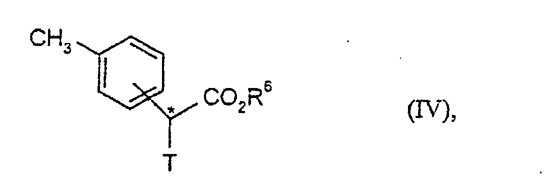 in which
T and R⁶ have the meaning specified,
then converting these, by halogenation, into the enantiomerically pure compounds of the general formula (V) 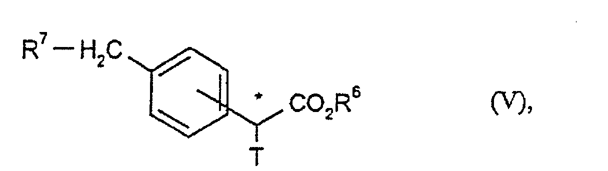 in which
T and R⁶ have the meaning specified
and
R⁷ represents halogen, such as chlorine, bromine, iodine, preferably bromine, reacting these in a further step with compounds of the general formula (VI)
A-H (VI)
in which
A has the meaning specified,
to give the enantiomerically pure compounds of the general formula (VII) 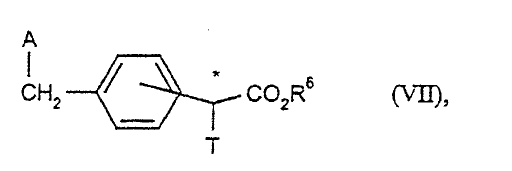 in which
A, T and R⁶ have the meaning specified,
and, in the case of compounds of the general formula (I) where Q = OH, carrying out a hydrolysis, and in the case where Q = activating radical, starting from the enantiomerically pure acids reacting with activating reagents.

2. Process according to Claim 1 for the preparation of compounds of the formula (I) according to Claim 1, in which
R¹ and R², including the double bond linking them, together form a phenyl ring or pyridyl ring or a ring of the formula 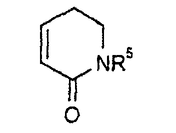 in which
R⁵ denotes hydrogen or linear or branched alkyl having up to 3 carbon atoms,
R³ and R⁴, including the double bond linking them, together form a phenyl ring or a cyclopentene, cyclohexene, cycloheptene, cyclooctene, oxocyclopentene, oxocyclohexene, oxocycloheptene or oxocyclooctene radical,
where all ring systems, listed under R¹/R² and R³/R⁴ are optionally up to disubstituted identically or differently by fluorine, chlorine, bromine, trifluoromethyl, carboxyl, hydroxyl, by linear or branched alkoxy or alkoxycarbonyl each having up to 4 carbon atoms, or by linear or branched alkyl having up to 4 carbon atoms, which itself can be substituted by hydroxyl or by linear or branched alkoxy having up to 3 carbon atoms,
T represents cyclobutyl, cyclopentyl, cyclohexyl, cycloheptyl, cyclooctyl, or represents linear or branched alkyl having up to 10 carbon atoms,
Q represents hydroxyl or represents an activating radical selected from the group consisting of chloride, bromide, mesylate, tosylate and triflate,
and their salts.

3. Process according to Claim l for the preparation of compounds of the formula (I) according to Claim 1 in which
R¹ and R², including the double bond linking them, together form a phenyl ring or pyridyl ring or a ring of the formula 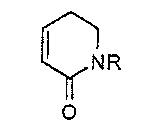 in which
R⁵ denotes hydrogen or methyl,
R³ and R⁴, including the double bond linking them, together form a phenyl ring or a cyclopentene, cyclohexene, cycloheptene, cyclooctene, oxocyclopentene, oxocyclohexene, oxocycloheptene or oxocyclooctene radical.

4. Process according to Claim 1 for the preparation of compounds of the general formula (I) according to Claim 1,
in which
A represents a radical of the formula 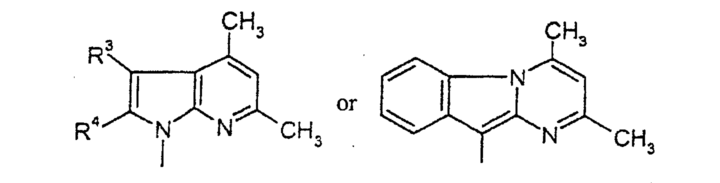
and
R³ and R⁴ = phenyl ring
and having the radical *CH-T-COQ in the paraposition and Q = chlorine.

5. Process according to one of Claims I to 4 in which compounds of the formula (II) are alkylated by compounds of the formula (III) in dimethylformamide with potassium tert-butanolate as base to give compounds of the formula (IV).

6. Process for preparing 2-(S)-2-cyclopentyl-2-[4-(2,4-dimethyl-a-carbolin-9-yl)-methyl]phenylacetic acid of the formula 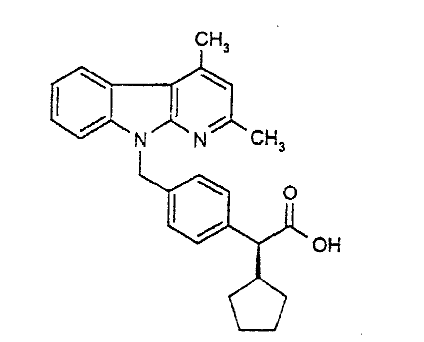 by converting:
(L)-menthyl 2-(4-tolyl)acetate of the formula 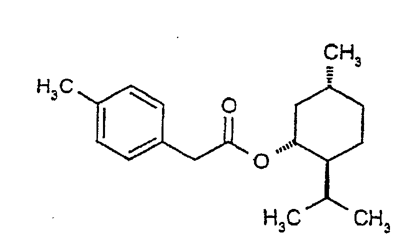 by cyclopentyl bromide in dimethylformamide in the presence of potassium tert-butanolate by diastereoselective alkylation into (L)-menthyl 2-(S)-2-cyclopentyl-2-(4-tolyl)acetate of the formula 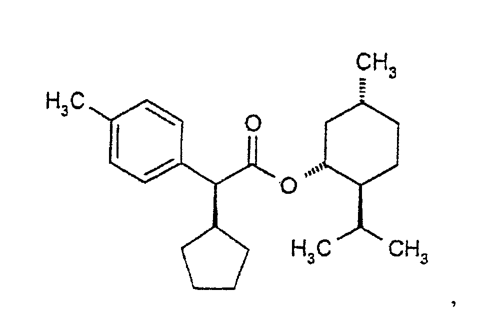 then converting this compound by halogenation into (L)-menthyl 2-(S)-2-(4-bromomethylphenyl)-2-cyclopentylacetate of the formula 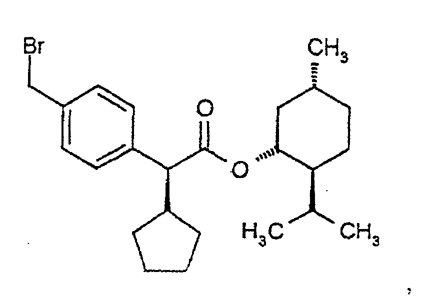 preparing from this compound and another step by reaction with α-carboline of the formula 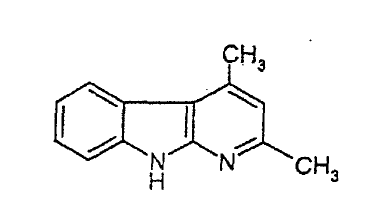
(L)-menthyl 2-(S)-2-cyclopentyl-2-[4-(2,4-dimethyl-α-carbolin-9-yl)methyl]-phenylacetate of the formula 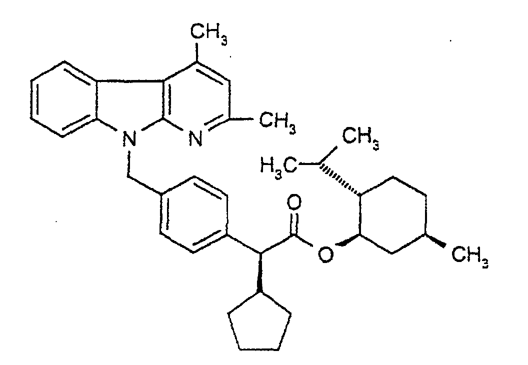 and hydrolysing this compound.

7. Process for the preparation of enantiomerically pure cycloalkanoindolecarboxylic acids and azaindolecarboxylic acids and pyrimido[1,2a]indolecarboxylic acids and their derivatives of the general formula (I) 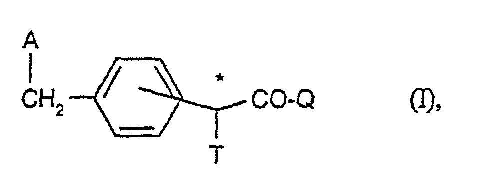 in which
A represents a radical of the formula 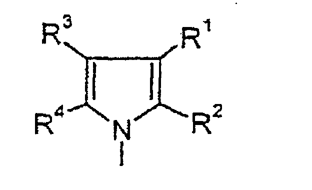 or 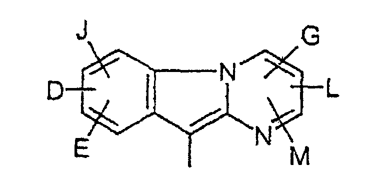
J, D, E, G, L and M are identical or different and denote hydrogen, halogen, trifluoromethyl, carboxyl, hydroxyl, linear or branched alkoxy or alkoxycarbonyl each having up to 6 carbon atoms or linear or branched alkyl having up to 6 carbon atoms, which itself can be substituted by hydroxyl or by linear or branched alkoxy having up to 4 carbon atoms,
in which
R¹ and R², including the double bond linking them, together form a phenyl ring or pyridyl ring or a ring of the formula 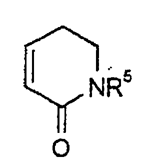
where
R⁵ denotes hydrogen or linear or branched alkyl having up to 4 carbon atoms,
R³ and R⁴, including the double bond linking them, together form a phenyl ring or a 4- to 8-membered cycloalkene or oxocycloalkene radical,
where all the ring systems listed under R¹/R² and R³/R⁴ are optionally up to trisubstituted identically or differently by halogen, trifluoromethyl, carboxyl, hydroxyl, by linear or branched alkoxy or alkoxycarbonyl each having up to 6 carbon atoms, or by linear or branched alkyl having up to 6 carbon atoms, which itself can be substituted by hydroxyl or by linear or branched alkoxy having up to 4 carbon atoms,
T represents cycloalkyl having 4 to 12 carbon atoms, or represents linear or branched alkyl having up to 12 carbon atoms,
Q represents hydroxyl or an activating radical selected from the group consisting of chloride, bromide, mesylate, tosylate and triflate,
and their salts,
**characterized in that** racemic carboxylic acids of the general formula (X) 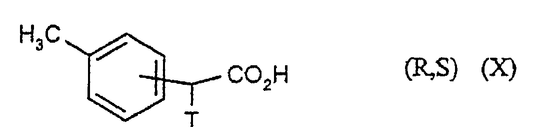 in which
T has the meaning specified above,
are first converted by reaction with (R)- or (S)-phenylethylamine in inert solvents and subsequent crystallization of the phenethylammonium salts and subsequent hydrolysis of the salts, into the enantiomerically pure compounds of the general formula (XI) 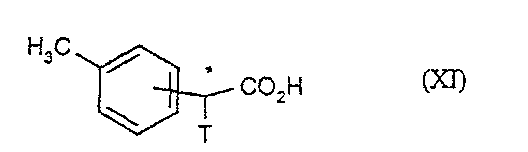 in which
T has the meaning specified above,
these are converted in a further step with isobutene, in inert solvents and in the presence of acids, into the enantiomerically pure esters (XII) 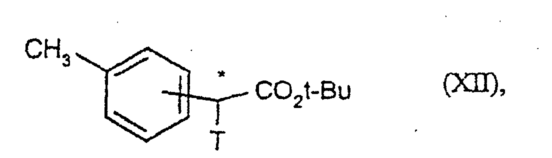 in which
T has the meaning specified above,
then the esters (XII) are converted by halogenation into the enantiomerically pure compounds of the general formula (XIII) 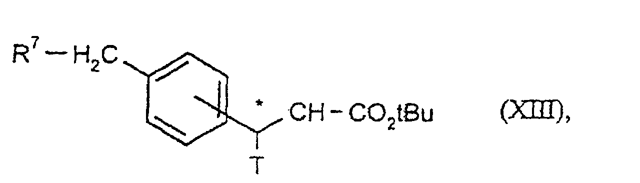 in which
R has the meaning specified above
and
R⁷ represents a typical leaving group, such as chlorine, bromine, iodine, tosylate or mesylate,
in a further step, by reaction with compounds of the general formula (VI)
A-H (VI)
in which
A has the meaning specified above,
the enantiomerically pure compounds of the general formula (I) 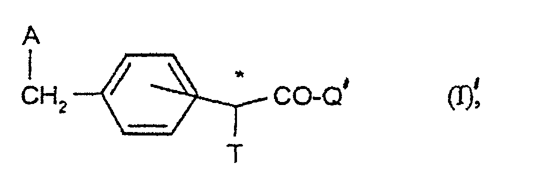 in which
A and T have the meaning specified above and
Q' represents tert-butyl,
are prepared,
and, in the case of the compounds of the general formula (I) where Q = OH, a hydrolysis is carried out.

8. Intermediates of the general formula 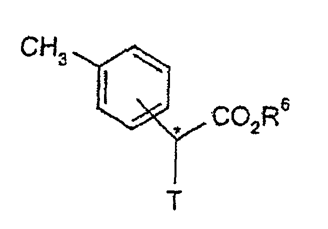 in which
T represents cycloalkyl having 4 to 12 carbon atoms or represents linear or branched alkyl having up to 12 carbon atoms
and
R⁶ represents the D- or L-menthyl radical or tert-butyl,
with the exception of the compound having T = isopropyl.

9. Intermediates of the general formula 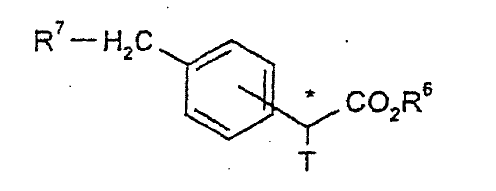 in which
R⁶ represents the D- or L-menthyl radical or represents the tert-butyl radical,
T represents cycloalkyl having 4 to 12 carbon atoms or represents linear or branched alkyl having up to 12 carbon atoms
and
R7 represents bromine.

10. Use of the intermediates according to Claims 8 and 9 for the preparation of enantiomerically pure active compounds of the formula (I) according to Claim 1,
in which
Q represents a phenylglycinol radical.

11. Intermediates of the general formula (I) 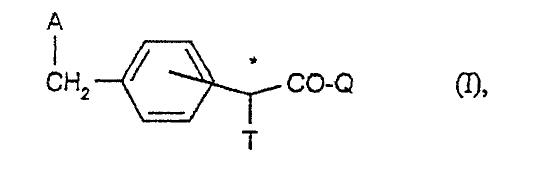 in which A represents 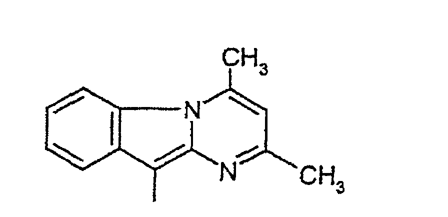 and T represents cycloalkyl having 4-12 carbon atoms or represents linear or branched alkyl having up to 12 carbon atoms and
Q represents hydroxyl or chlorine and their salts.

12. Intermediates of the general formula (VII) 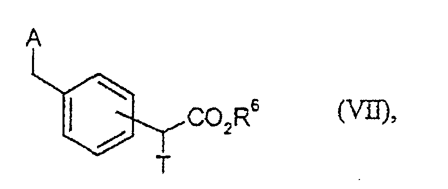 in which A represents 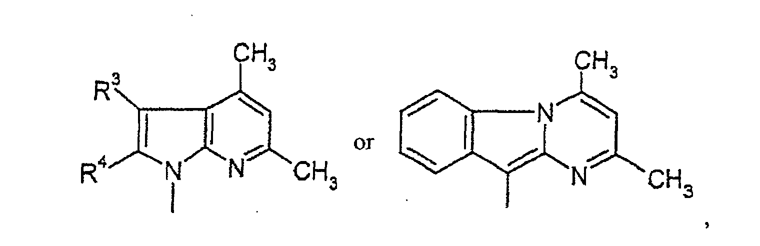
R³ and R⁴ have the meaning specified above,
T represents cycloalkyl having 4 to 12 carbon atoms or linear or branched alkyl having up to 12 carbon atoms and
R6 represents D- or L-menthyl.

13. Intermediates of the group consisting of:
a) Tert-butyl (S)-2-(4-bromomethylphenyl)-2-cyclopentyl-acetate of the formula 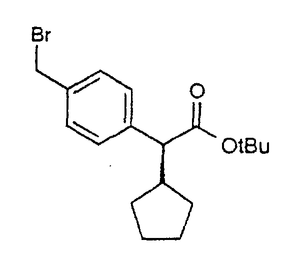
c) (L)-menthyl 2-(S)-2-(4-bromomethyl-phenyl)-2-cyclopentyl-acetate of the formula 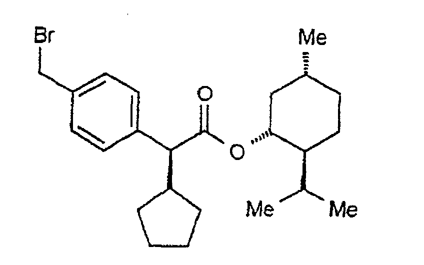
d) L-menthyl 2(S)-2-cyclopentyl-2[4-(2,4-dimethyl-a-carbolin-9-yl)methyl]-phenylacetate of the formula 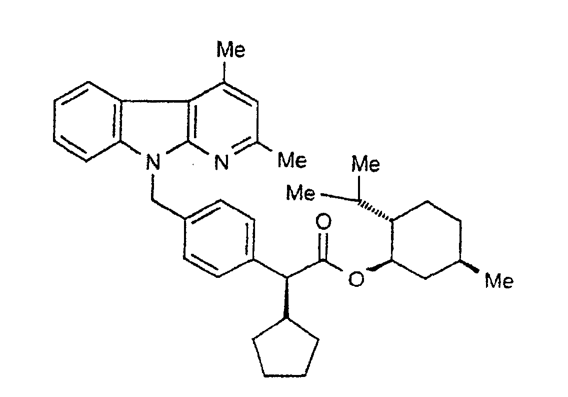
e) 2-(S)-2-cyclopentyl-2-[4-(2,4-dimethyl-α-carbolin-9-yl)methyl]phenylacetyl chloride of the formula 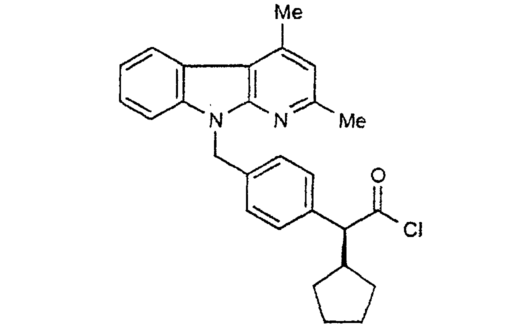
f) L-menthyl 2-(S)-2-cyclopentyl-2-[4-(2,4-dimethyl-pyrimido[1,2-a]-indol-9-yl)-methyl)phenyl] acetate of the formula 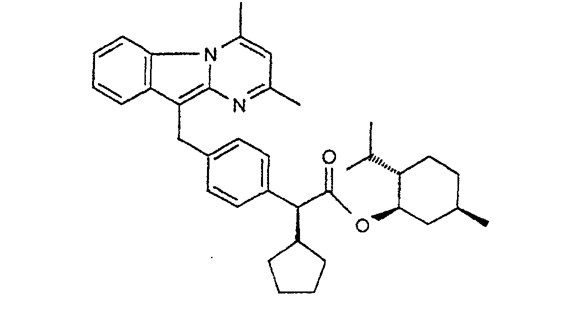
g) menthyl 2-(S)-2-cyclopentyl-2-[4-(2,4-dimethyl-pyrimido[1,2-a]-indol-9-yl)-methyl)phenyl]acetate hydrochloride of the formula 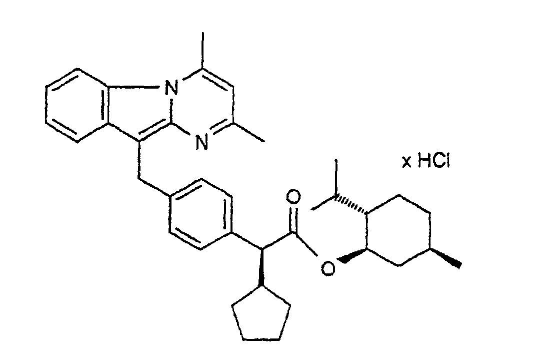
h) 2-(S)-2-cyclopentyl-2-[4-(2,4-dimethyl-pyrimido[1,2-a]-indo]-9-yl)-methy])pheny]]acety] chloride of the formula

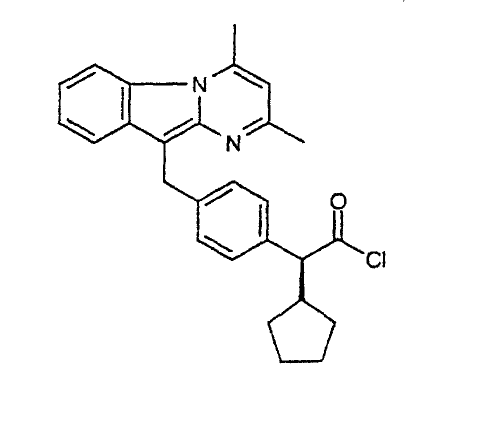

## Revendications

1. Procédé de production d'acides cyclo-alcano-indole- et aza-indole-carboxyliques et d'acides pyrimido[1,2a]-indole-carboxyliques énantiomères purs et de dérivés activés de ces acides, de formule générale (I) 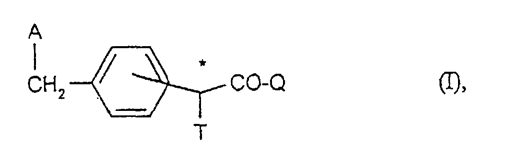 dans laquelle
A représente un reste de formule 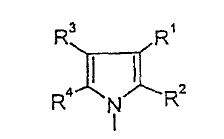 ou 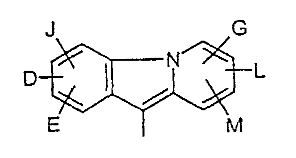
J, D, E, G, L et M sont identiques ou différents et représentent l'hydrogène, un halogène, un reste trifluorométhyle, carboxy, hydroxy, un reste alkoxy ou alkoxycarbonyle linéaire ou ramifié ayant chacun jusqu'à 6 atomes de carbone ou un reste alkyle linéaire ou ramifié ayant jusqu'à 6 atomes de carbone, qui peut lui-même être substitué par un radical hydroxy ou par un radical alkoxy linéaire ou ramifié ayant jusqu'à 4 atomes de carbone,
où
R¹ et R² forment ensemble conjointement avec la double liaison qui les relie un noyau phényle ou pyridyle ou un noyau de formule 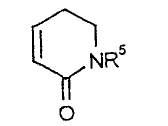 dans laquelle
R⁵ représente l'hydrogène ou un reste alkyle linéaire ou ramifié ayant jusqu'à 4 atomes de carbone,
R³ et R⁴ forment ensemble conjointement avec la double liaison qui les relie un noyau phényle ou un reste cyclo-alcène ou oxo-cyclo-alcène de 4 à 8 chaînons,
tous les systèmes de noyaux indiqués pour R¹/R² et R³/R⁴ étant éventuellement substitués jusqu'à trois fois identiques ou différentes par un halogène, un reste trifluorométhyle, carboxy, hydroxy, par un reste alkoxy ou alkoxycarbonyle linéaire ou ramifié ayant chacun jusqu'à 6 atomes de carbone ou par un reste alkyle linéaire ou ramifié ayant jusqu'à 6 atomes de carbone, qui peut lui-même être substitué par un radical hydroxy ou par un radical alkoxy linéaire ou ramifié ayant jusqu'à 4 atomes de carbone,
T est un reste cycloalkyle ayant 4 à 12 atomes de carbone ou un reste alkyle linéaire ou ramifié ayant jusqu'à 12 atomes de carbone,
Q est un groupe hydroxy ou un reste activateur choisi dans le groupe des restes chlorure, bromure, mésylate, tosylate et triflate,
et de leurs sels,
dans lequel on transforme des composés de formule générale (II) 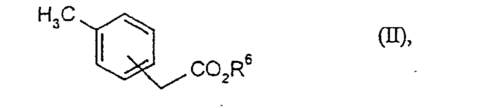 dans laquelle
R⁶ forme conjointement avec l'atome d'oxygène un reste alcool chiral choisi dans le groupe des restes (+)- ou (-) -menthyle, (+) - ou (-)bornyle, (+)- ou (-)-iso-bornyle ou (-)-8-phénylmenthyle, tout d'abord avec des composés de formule générale (III)
T-Z (III)
dans laquelle
T a la défniition indiquée et
Z est un groupe partant typique tel que, par exemple, brome, chlore, iode, mésyle, tosyle ou trifluorométhylsulfonyle, de préférence iode et brome,
dans des solvants inertes en présence d'une base, par alkylation diastéréosélective, en les composés énantiomères purs de formule générale (IV) 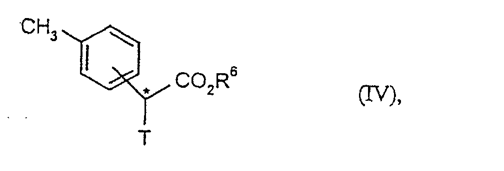 dans laquelle
T et R⁶ ont la définition indiquée,
on transforme ensuite ces composés par halogénation en les composés énantiomères purs de formule générale (V) 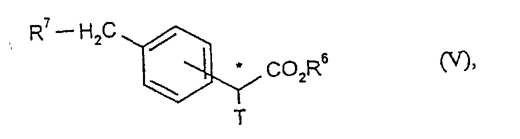 dans laquelle
T et R⁶ ont la définition indiquée
et
R⁷ est un halogène tel que, par exemple, le chlore, le brome ou l'iode, de préférence le brome,
on prépare à partir de ces composés dans une autre étape , par réaction avec des composés de formule générale (VI)
A-H (VI)
dans laquelle
A a la définition indiquée,
les composés énantiomères purs de formule générale (VII) 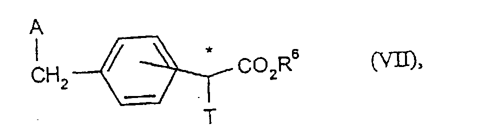 dans laquelle
A, T et R⁶ ont la définition indiquée,
et dans le cas de composés de formule générale (I) dans laquelle Q représente OH, on conduit une hydrolyse et au cas où Q représente un reste activateur, on conduit une réaction à partir des acides énantiomères purs avec des réactifs activateurs.

2. Procédé suivant la revendication 1, pour la production de composés de formule (I) suivant la revendication 1, dans laquelle
R¹ et R² forment ensemble conjointement avec la double liaison qui les relie un noyau phényle ou pyridyle ou un noyau de formule 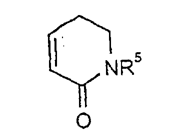 dans laquelle
R⁵ représente l'hydrogène ou un reste alkyle linéaire ou ramifié ayant jusqu'à 3 atomes de carbone,
R³ et R⁴ forment ensemble conjointement avec la double liaison qui les relie un noyau phényle ou un noyau cyclopentène, cyclohexène, cycloheptène, cyclo-octène, oxocyclopentène, oxocyclohexène, oxocycloheptène ou oxocyclo-octène,
tous les systèmes de noyaux indiqués pour R¹/R² et R³/R⁴ étant éventuellement substitués jusqu'à deux fois identiques ou différentes par du fluor, du chlore, du brome, un reste trifluorométhyle, carboxy, hydroxy, par un reste alkoxy ou alkoxycarbonyle linéaire ou ramifié ayant chacun jusqu'à 4 atomes de carbone ou par un reste alkyle linéaire ou ramifié ayant jusqu'à 4 atomes de carbone, qui peut lui-même être substitué par un radical hydroxy ou par un radical alkoxy linéaire ou ramifié ayant jusqu'à 3 atomes de carbone,
T est un reste cyclobutyle, cyclopentyle, cyclohexyle, cycloheptyle, cyclo-octyle ou un reste alkyle linéaire ou ramifié ayant jusqu'à 10 atomes de carbone,
Q est un groupe hydroxy ou un reste activateur choisi dans le groupe des restes chlorure, bromure, mésylate, tosylate, triflate,
et de leurs sels.

3. Procédé suivant la revendication 1, pour la production de composés de formule (I) suivant la revendication 1,
dans laquelle
R¹ et R² forment ensemble conjointement avec la double liaison qui les relie un noyau phényle ou pyridyle ou un noyau de formule 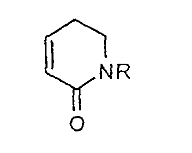 dans laquelle
R⁵ représente l'hydrogène ou un reste méthyle,
R³ et R⁴ forment ensemble conjointement avec la double liaison qui les relie un noyau phényle ou un reste cyclo-pentène, cyclohexène, cycloheptène, cyclo-octène, oxocyclo-pentène, oxycyclohexène, oxocycloheptène ou oxocyclo-octène.

4. Procédé suivant la revendication 1, pour la production de composés de formule générale (I) suivant la revendication 1,
où
A est un reste de formule 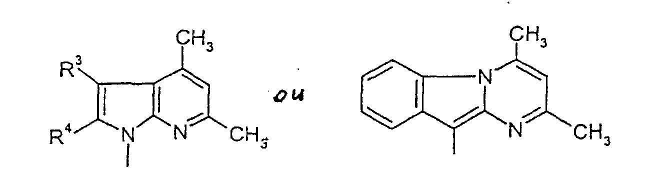 et
R³ et R⁴ représentent un noyau phényle
et avec le reste *CH-T-COQ en position para et Q représentant le chlore.

5. Procédé suivant l'une des revendications 1 à 4, dans lequel l'alkylation de composés de formule (II) en composés de formule (IV) avec des composés de formule (III) est conduite dans le diméthylformamide avec le tertio-butanolate de potassium comme base.

6. Procédé de production de l'acide 2-(S)-2-cyclopentyl-2-[4-(2,4-diméthyl-α-carboline-9-yl)-méthyl]phénylacétique de formule 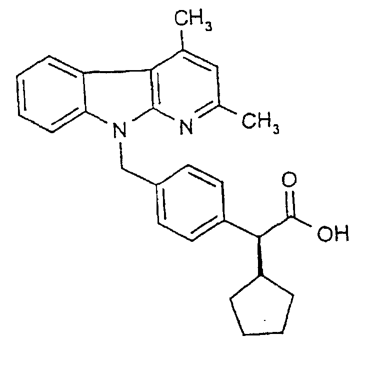 dans lequel
on transforme l'ester de (L)-mentyle de l'acide 2-(4-tolyl)-acétique de formule 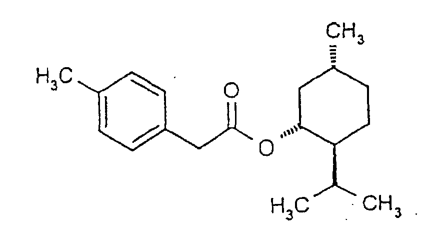 avec le bromure de cyclopentyle dans le diméthylformamide, en présence de tertio-butanolate de potassium, par alkylation diastéréosélective en l'ester de (L)-menthyle d'acide 2-(S)-2-cyclopentyl-2-(4-tolyl)-acétique de formule 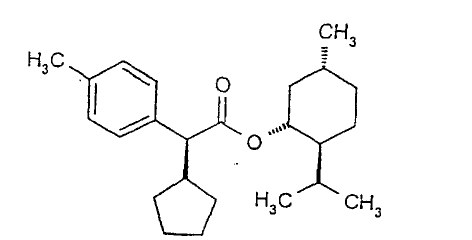 on transforme ensuite ce composé par halogénation en l'ester de (L)-menthyle d'acide 2-(S)-2-(4-bromométhylphényl)-2-cyclopentyl-acétique de formule 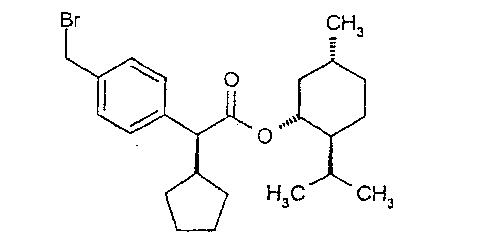 on prépare à partir de ce composé dans une autre étape par réaction avec l'α-carboline de formule 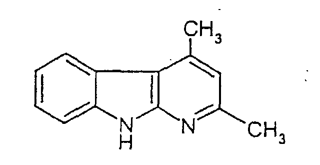 l'ester de (L)-menthyle d'acide 2- (S)-2-cyclopentyl-2-[4-(2,4-diméthyl-α-carboline-9-yl)-méthyl]-phényl-acétique de formule 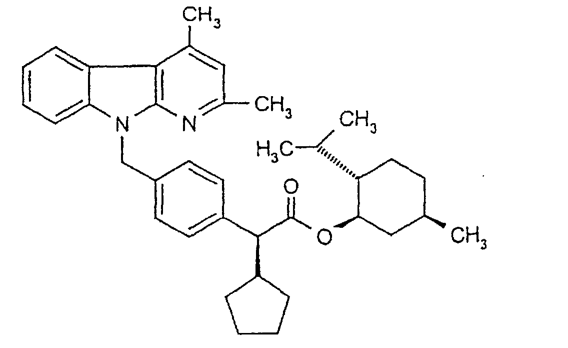 et on hydrolyse ce composé.

7. Procédé de production d'acides cyclo-alcano-indole- et aza-indole- et pyrimido[1,2a]indole-carboxyliques énantiomères purs et de leurs dérivés, de formule générale (I) 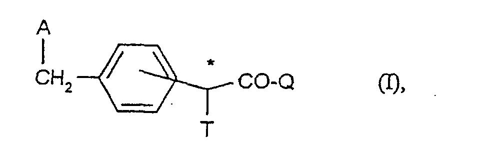 dans laquelle
A représente un reste de formule 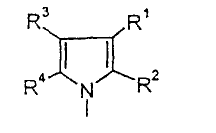 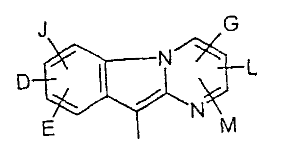
J, D, E, G, L et M sont identiques ou différents et représentent l'hydrogène, un halogène, un reste trifluorométhyle, carboxy, hydroxy, un reste alkoxy ou alkoxycarbonyle linéaire ou ramifié ayant chacun jusqu'à 6 atomes de carbone ou un reste alkyle linéaire ou ramifié ayant jusqu'à 6 atomes de carbone, qui peut lui-même porter un substituant hydroxy ou un substituant alkoxy linéaire ou ramifié ayant jusqu'à 4 atomes de carbone,
où
R¹ et R² forment ensemble conjointement avec la double liaison qui les relie un noyau phényle ou pyridyle ou un noyau de formule 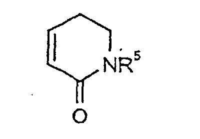 dans laquelle
R⁵ est l'hydrogène ou un reste alkyle linéaire ou ramifié ayant jusqu'à 4 atomes de carbone,
R³ et R⁴ forment ensemble conjointement avec la double liaison qui les relie un noyau phényle ou un reste cyclo-alcène ou oxocyclo-alcène de 4 à 8 chaînons,
tous les systèmes de noyaux indiqués pour R¹/R² et R³/R⁴ étant éventuellement substitués jusqu'à trois fois identiques ou différentes par un halogène, un reste trifluorométhyle, carboxy, hydroxy, par un reste alkoxy ou alkoxycarbonyle linéaire ou ramifié ayant chacun jusqu'à 6 atomes de carbone ou par un reste alkyle linéaire ou ramifié ayant jusqu'à 6 atomes de carbone, qui peut lui-même être substitué par un radical hydroxy ou par un radical alkoxy linéaire ou ramifié ayant jusqu'à 4 atomes de carbone,
T est un reste cycloalkyle ayant 4 à 12 atomes de carbone ou un reste alkyle linéaire ou ramifié ayant jusqu'à 12 atomes de carbone,
Q est un groupe hydroxy ou un reste activateur choisi dans le groupe des restes chlorure, bromure, mésylate, tosylate, triflate, et de leurs sels,
**caractérisé en ce qu'**on transforme tout d'abord des acides carboxyliques racémiques de formule générale (X) 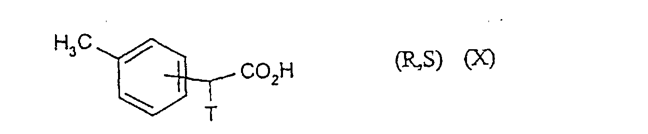 dans laquelle
T a la définition indiquée ci-dessus,
par réaction avec la (R)- ou la (S)-phényléthylamine dans des solvants inertes suivie de la cristallisation des sels de phénéthylammonium, puis de l'hydrolyse des sels, en les composés énantiomères purs de formule générale (XI) 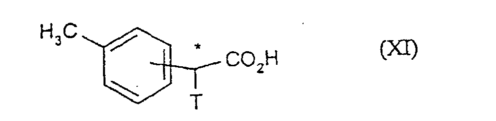 dans laquelle
T a la définition indiquée ci-dessus,
on transforme ces composés dans une autre étape avec l'isobutène dans des solvants inertes et en présence d'acides, en les esters énantiomères purs de formule (XII) 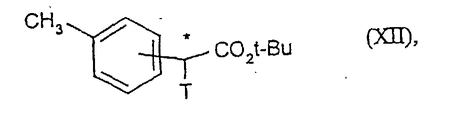 dans laquelle
T a la définition indiquée ci-dessus,
puis on transforme les esters (XII) par halogénation en les composés énantiomères purs de formule générale (XIII) 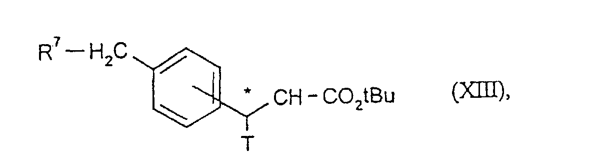 dans laquelle
R a la définition indiquée ci-dessus
et
R⁷ représente un groupe partant typique tel que, par exemple, chlore, brome, iode, tosylate ou mésylate,
on prépare dans une autre étape par réaction avec des composés de formule générale (VI)
A-H (VI)
dans laquelle
A a la définition indiquée ci-dessus,
les composés énantiomères purs de formule générale (I) 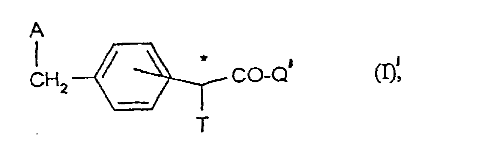 dans laquelle
A et T ont la définition indiquée ci-dessus et
Q¹ est un groupe tertio-butyle,
et dans le cas de composés de formule générale (I) dans laquelle Q est un groupe OH, on effectue une hydrolyse.

8. Produits intermédiaires de formule générale 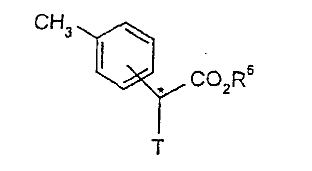 dans laquelle
T est un reste cycloalkyle ayant 4 à 12 atomes de carbone ou un reste alkyle linéaire ou ramifié ayant jusqu'à 12 atomes de carbone
et
R⁶ est un reste D- ou L-menthyle ou un reste tertiobutyle,
le composé dans lequel T est un reste isopropyle étant exclu.

9. Produits intermédiaires de formule générale 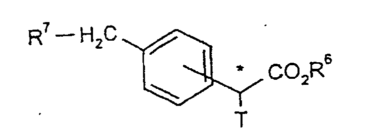 dans laquelle
R⁶ est un reste D- ou L-menthyle ou le reste tertiobutyle,
T est un reste cycloalkyle ayant 4 à 12 atomes de carbone ou un reste alkyle linéaire ou ramifié ayant jusqu'à 12 atomes de carbone
et
R⁷ est le brome.

10. Utilisation des produits intermédiaires suivant les revendications 8 et 9 pour la préparation de substances actives énantiomères pures de formule (I) suivant la revendication 1
où
Q est un reste phénylglycinol.

11. Produits intermédiaires de formule générale (I) 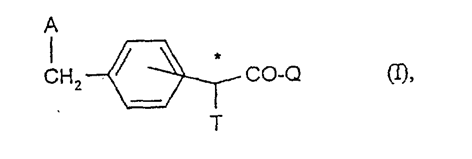 dans laquelle A est un reste 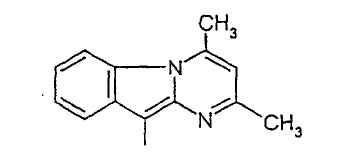 et T est un reste cycloalkyle ayant 4 à 12 atomes de carbone ou un reste alkyle linéaire ou ramifié ayant jusqu'à 12 atomes de carbone, et
Q est un groupe hydroxy ou le chlore, et leurs sels.

12. Produits intermédiaires de formule générale (VII) 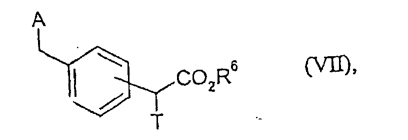 dans laquelle
A est un reste 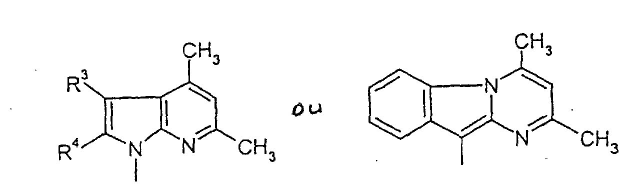 steht,
R³ et R⁴ ont la définition indiquée ci-dessus,
T est un reste cycloalkyle ayant 4 à 12 atomes de carbone ou un reste alkyle linéaire ou ramifié ayant jusqu'à 12 atomes de carbone et
R⁶ est un reste D- ou L-menthyle.

13. Composés intermédiaires de la série :
a) ester tertio-butylique d'acide (S)-2-(4-bromométhylphényl)-2-cyclopentyl-acétique de formule 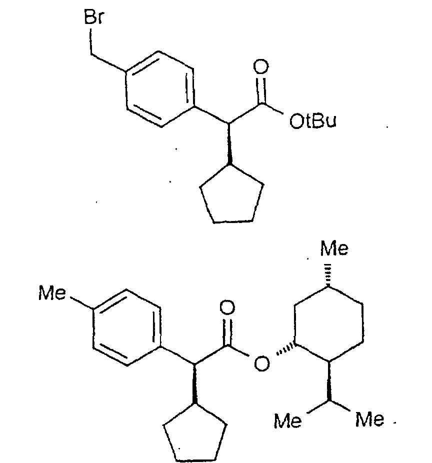
c) ester de (L)-menthyle de l'acide 2-(S)-2-(4-bromométhylphényl)-2-cyclopentyl-acétique de formule 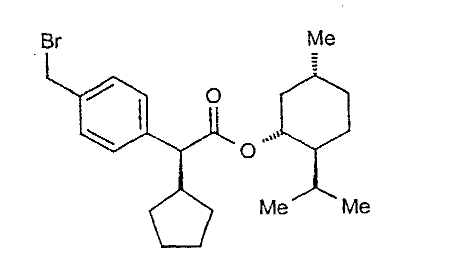
d) ester de L-menthyle de l'acie 2-(S)-2-cyclopentyl-2-[4-(2,4-diméthyl-a-carboline-9-yl)méthyl]phényl-acétique de formule 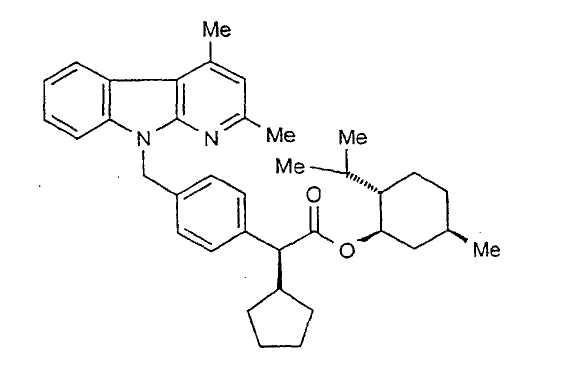
e) chlorure d'acide 2-(S)-2-cyclopentyl-2-[4-(2,4-diméthyol-α-carboline-9-yl)méthyl]phényl-acétique de formule 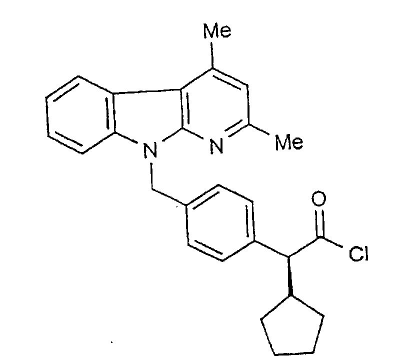
f) ester de L-menthyle de l'acide 2-(S)-2-cyclopentyl-2-[4-(2,4-diméthylpyrimido[1,2-a]-indole-9-yl)méthyl)-phényl]-acétique de formule 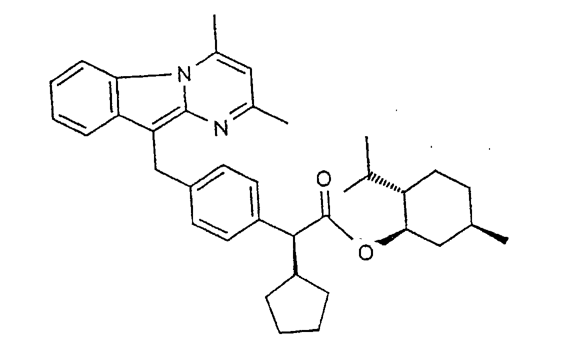
g) chlorhydrate d'ester de menthyle de l'acide 2-(S)-2-cyclopentyl-2-[4-(2,4-diméthylpyrimido[1,2-a]-indole-9-yl)méthyl)phényl]-acétique de formule 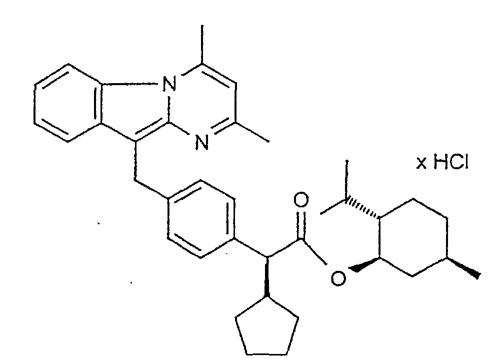
h) chlorure d'acide 2-(S)-2-cyclopentyl-2-[4-(2,4-diméthylpyrimido[1,2-a]-indole-9-yl)méthyl)phényl]-acétique de formule
